# EUROPEAN PATENT APPLICATION

(11) **EP 4 850 668 A1**
(43) Date of publication of application: **09.12.2026**
(21) Application number: 25748041.8
(22) Date of filing: 04.02.2025
(51) Int. Cl.: A61K 31/343, A61K 31/704, A61P 9/10, A61P 25/16, A61P 25/28

(54) **MEDICAMENT FOR TREATING BRAIN DISEASES**

(30) Priority: 04.02.2024 CN 202410159181
(71) Applicant: Shanghai Institute of Materia Medica, Chinese Academy of Sciences, Shanghai 201203 (CN)
(72) Inventor: JIANG, Baohong, Shanghai 201203 (CN); WANG, Linlin, Shanghai 201203 (CN); SHEN, Haishang, Shanghai 201203 (CN); ZHANG, Yuhan, Shanghai 201203 (CN); CHEN, Siqi, Shanghai 201203 (CN); WANG, Yiyu, Shanghai 201203 (CN); ZHANG, Lingxiao, Shanghai 201203 (CN)
(74) Representative: Mewburn Ellis LLP
(86) International application number: PCT/CN2025/075654
(87) International publication number: WO 2025/162479

(57) **Abstract**

The present disclosure provides a medicament for treating brain diseases. Specifically, provided is a pharmaceutical composition. The active ingredient of the pharmaceutical composition comprises: (a) a first active ingredient, wherein the first active ingredient is a pharmaceutically acceptable salt of salvianolic acid; and (b) a second active ingredient, wherein the second active ingredient is ginsenoside or a pharmaceutically acceptable salt thereof. The pharmaceutically acceptable salt of salvianolic acid is selected from the following group: magnesium salvianolate B, calcium salvianolate B, magnesium salvianolate A, sodium rosmarinate, and a combination thereof. The ginsenoside is selected from the following group: ginsenoside Rg1, ginsenoside Rh2, ginsenoside Rb1, and a combination thereof. The mass ratio of the first active ingredient to the second active ingredient is 1:(0.1-10), wherein the mass ratio is based on the pharmaceutically acceptable salt of salvianolic acid and ginsenoside. Also provided is use of the pharmaceutical composition in preventing or treating brain diseases.

## Description

### Technical Field

The present invention relates to the field of the pharmaceutical industry, specifically to a medicament for treating brain diseases and applications thereof.

### Background Art

Brain diseases are diseases that cause abnormal brain function due to various reasons. The most common brain diseases are cerebrovascular diseases, including cerebral arteriosclerosis, cerebral circulation insufficiency, ischemic stroke, cerebral hemorrhage, etc.; and neurodegenerative diseases of the nervous system, including senile dementia, Parkinson's disease, multiple system atrophy, progressive supranuclear palsy, etc.

As a vital central nervous system organ, the brain controls various physiological and psychological activities of the human body. Patients with the above-mentioned brain diseases suffer from varying degrees of impairment in bodily functions such as language and walking abilities, and most have disabling sequelae to varying degrees, such as hemiplegia, slurred speech, mental retardation, joint stiffness and contractures, and even dementia, which seriously affects the patient's quality of life and places a heavy burden on their families.

Therefore, there is a need in the art to provide more candidate drugs with superior efficacy for preventing or treating brain diseases.

### Summary of the Invention

The purpose of the present invention is to provide a medicament that can prevent or treat brain diseases.

In a first aspect, the present invention provides a pharmaceutical composition, wherein the active ingredients of the pharmaceutical composition comprise (a) and (b):
(a) a first active ingredient, which is a pharmaceutically acceptable salt of salvianolic acid; and
(b) a second active ingredient, which is ginsenoside, or a pharmaceutically acceptable salt thereof;
and the weight ratio of the first active ingredient to the second active ingredient is 1:(0.1-10), wherein the weight ratio is calculated based on the pharmaceutically acceptable salt of salvianolic acid and ginsenoside.

In another preferred embodiment, the first active ingredient is a pharmaceutically acceptable salt of salvianolic acid B; and the second active ingredient is ginsenoside Rg1, or a pharmaceutically acceptable salt thereof;
and the weight ratio of the first active ingredient to the second active ingredient is 1:(0.1-10), wherein the weight ratio is calculated based on the pharmaceutically acceptable salt of salvianolic acid B and ginsenoside Rg1.

In another preferred embodiment, the pharmaceutically acceptable salt of salvianolic acid is selected from the group consisting of: calcium salt, magnesium salt, sodium salt, potassium salt, lithium salt, ammonium salts (e.g., diethylamine, trimethylamine, diethanolamine), piperazine salt, zinc salt, silver salt, amino acid salts, or combinations thereof, preferably a magnesium salt.

In another preferred embodiment, the active ingredients of the pharmaceutical composition comprise (a) and (b):
(a) a first active ingredient, which is a pharmaceutically acceptable salt of salvianolic acid; and
(b) a second active ingredient, which is ginsenoside, or a pharmaceutically acceptable salt thereof;

wherein the pharmaceutically acceptable salt of salvianolic acid is selected from the group consisting of: magnesium salvianolate B, calcium salvianolate B, magnesium salvianolate A, sodium rosmarinate, or combinations thereof;
the ginsenoside is selected from the group consisting of: ginsenoside Rg1, ginsenoside Rh2, ginsenoside Rb1, or combinations thereof;
and the weight ratio of the first active ingredient to the second active ingredient is 1:(0.1-10), wherein the weight ratio is calculated based on the pharmaceutically acceptable salt of salvianolic acid and ginsenoside.

In another preferred embodiment, the first active ingredient is a pharmaceutically acceptable salt of salvianolic acid B; and (b) a second active ingredient, which is ginsenoside, or a pharmaceutically acceptable salt thereof;
wherein the pharmaceutically acceptable salt of salvianolic acid B is selected from the group consisting of: magnesium salvianolate B, calcium salvianolate B, or a combination thereof;
and the weight ratio of the first active ingredient to the second active ingredient is 1:(0.1-10), wherein the weight ratio is calculated based on the pharmaceutically acceptable salt of salvianolic acid B and ginsenoside. Preferably, the pharmaceutical composition further comprises a third active ingredient: other salvianolic acids, other ginsenosides, or combinations thereof, such as magnesium salvianolate A, sodium rosmarinate, ginsenoside Rb1, ginsenoside Rh1, or combinations thereof.

In another preferred embodiment, the first active ingredient is magnesium salvianolate B.

In another preferred embodiment, the purity of the pharmaceutically acceptable salt of salvianolic acid B is ≥ 80%, preferably ≥ 85%, more preferably ≥ 90%, ≥ 95%, 98%, ≥ 99% or 99.5%, by weight.

In another preferred embodiment, the pharmaceutical composition further comprises a second active ingredient: ginsenoside Rg1, or a pharmaceutically acceptable salt thereof.

In another preferred embodiment, the first active ingredient is magnesium salvianolate B, and (b) a second active ingredient, which is ginsenoside Rg1, or a pharmaceutically acceptable salt thereof;
and the weight ratio of the first active ingredient to the second active ingredient is 1:(0.1-10), wherein the weight ratio is calculated based on magnesium salvianolate B and ginsenoside Rg1.

In another preferred embodiment, the first active ingredient is magnesium salvianolate B, and (b) a second active ingredient, which is ginsenoside Rg1;
and the weight ratio of the first active ingredient to the second active ingredient is 1:(0.1-10), wherein the weight ratio is calculated based on magnesium salvianolate B and ginsenoside Rg1.

In another preferred embodiment, the first active ingredient is a pharmaceutically acceptable salt of salvianolic acid A; and the second active ingredient is ginsenoside Rb1, or a pharmaceutically acceptable salt thereof;
and the weight ratio of the first active ingredient to the second active ingredient is 1:(0.1-10), wherein the weight ratio is calculated based on the pharmaceutically acceptable salt of salvianolic acid A and ginsenoside Rb1, preferably magnesium salvianolate A and ginsenoside Rb1 or ginsenoside Rh1.

In another preferred embodiment, the first active ingredient is a pharmaceutically acceptable salt of rosmarinic acid; and the second active ingredient is ginsenoside Rb1, or a pharmaceutically acceptable salt thereof;
and the weight ratio of the first active ingredient to the second active ingredient is 1:(0.1-10), wherein the weight ratio is calculated based on the pharmaceutically acceptable salt of rosmarinic acid and ginsenoside Rb1, preferably sodium rosmarinate and ginsenoside Rb1.

In another preferred embodiment, the weight ratio of the first active ingredient to the second active ingredient is 1:(0.2-5), preferably 1:(0.4-0.6) or 1:(2-3).

In another preferred embodiment, the weight ratio of the first active ingredient to the second active ingredient is 1:(0.3-0.5), preferably 1:(0.35-0.45), more preferably 1:(0.38-0.42), even more preferably 1:(0.39-0.41), or 1:0.4.

In another preferred embodiment, the weight ratio of the first active ingredient to the second active ingredient is 1:0.1, 1:0.15, 1:0.2, 1:0.25, 1:0.3, 1:0.35, 1:0.38, 1:0.39, 1:0.4, 1:0.41, 1:0.42, 1:0.45, 1:0.5, 1:0.55, 1:0.6, 1:0.65, 1:0.7, 1:0.75, 1:0.8, 1:0.85, 1:0.9, 1:0.95, 1:1, 1:1.2, 1:1.4, 1:1.5, 1:1.6, 1:1.8, 1:2, 1:2.2, 1:2.4, 1:2.5, 1:2.6, 1:2.8, 1:3, 1:3.2, 1:3.5, 1:4, 1:4.5, 1:5, 1:5.5, 1:6, 1:6.5, 1:7, 1:7.5, 1:8, 1:8.5, 1:9, 1:9.5 or 1:10.

In another preferred embodiment, the pharmaceutical composition further comprises (c) a pharmaceutically acceptable carrier.

In another preferred embodiment, the purity of the pharmaceutically acceptable salt of salvianolic acid B in the first active ingredient is ≥ 80%, preferably ≥ 90%, more preferably ≥ 95%, 98%, ≥ 99% or 99.5%.

In another preferred embodiment, the purity of ginsenoside Rg1 in the second active ingredient is ≥ 80%, preferably ≥ 90%, more preferably ≥ 95%, 98%, ≥ 99% or 99.5%.

In another preferred embodiment, the dosage form of the pharmaceutical composition is selected from the group consisting of: liquid preparations (e.g., solutions, emulsions, suspensions), solid preparations (e.g., lyophilized preparations), gaseous dosage forms, semi-solid dosage forms.

In another preferred embodiment, the dosage form is selected from the group consisting of: injections (e.g., injection solutions or powders for injection), oral preparations (e.g., capsules, tablets, pills, powders, granules, syrups, oral liquids or tinctures), sublingual preparations (e.g., lozenges, drops), respiratory tract administration preparations, skin administration preparations, mucosal administration preparations, more preferably, the dosage form is injections.

In another preferred embodiment, the pharmaceutical composition is a pharmaceutical composition for oral administration.

In another preferred embodiment, the dosage form of the pharmaceutical composition for oral administration is an orally disintegrating tablet, mucoadhesive tablet, oral dissolving film, orally disintegrating granule, powders, sublingual tablet, buccal tablet, chewable tablet, effervescent tablet, or solutions.

In a second aspect, the present invention provides a pharmaceutically acceptable salt of salvianolic acid B, wherein the salt is magnesium salvianolate B or calcium salvianolate B.

In another preferred embodiment, the pharmaceutically acceptable salt of salvianolic acid B is (mono-)magnesium salvianolate B.

In another preferred embodiment, the purity of the pharmaceutically acceptable salt of salvianolic acid B is ≥ 80%, ≥ 85%, preferably ≥ 90%, more preferably ≥ 95%, 98%, ≥ 99% or 99.5%, by weight.

In a third aspect, the present invention provides a method for preparing magnesium salvianolate B, comprising the step: in water or an aqueous solvent, reacting free salvianolic acid B with an inorganic salt or inorganic base containing magnesium ions, thereby generating magnesium salvianolate B.

In another preferred embodiment, the inorganic salt or inorganic base containing magnesium ions is selected from the group consisting of: magnesium carbonate, magnesium sulfate, magnesium nitrate, magnesium hydroxide, magnesium chloride, magnesium bicarbonate, or combinations thereof.

In another preferred embodiment, the method comprises the steps: adding an aqueous solution of free salvianolic acid B to an aqueous solution of an inorganic salt or inorganic base containing magnesium ions, thereby reacting to generate magnesium salvianolate B.

In another preferred embodiment, the molar ratio of free salvianolic acid to the inorganic salt or inorganic base containing magnesium ions is 1:0.98-1.02, preferably 1:0.99-1.01, more preferably 1:1.

In another preferred embodiment, the method further comprises steps of: loading the above reaction solution onto a C18 reverse-phase chromatographic column, performing gradient elution sequentially with pure water, 10% methanol-water, 15% methanol-water solution, and 20% methanol-water solution, collecting fractions, combining fractions with acceptable purity, removing methanol, re-loading the methanol-removed sample onto a C18 reverse-phase chromatographic column, performing gradient elution sequentially with pure water, 10% methanol-water, and 20% methanol-water solution, combining fractions with acceptable purity, and drying, thereby obtaining magnesium salvianolate B.

In a fourth aspect, the present invention provides a method for preparing calcium salvianolate B, comprising the step: in water or an aqueous solvent, reacting free salvianolic acid B with an inorganic salt or inorganic base containing calcium ions, thereby generating calcium salvianolate B.

In another preferred embodiment, the inorganic salt or inorganic base containing calcium ions is selected from the group consisting of: calcium carbonate, calcium sulfate, calcium nitrate, calcium hydroxide, calcium bicarbonate, or combinations thereof.

In another preferred embodiment, the method comprises a step of: adding an aqueous solution of free salvianolic acid B to an aqueous solution of an inorganic salt or inorganic base containing calcium ions, thereby reacting to generate calcium salvianolate B.

In another preferred embodiment, the molar ratio of free salvianolic acid to the inorganic salt or inorganic base containing calcium ions is 1:0.98-1.02, preferably 1:0.99-1.01, more preferably 1:1.

In another preferred embodiment, the method further comprises steps of: loading the above reaction solution onto a C18 reverse-phase chromatographic column, performing gradient elution sequentially with pure water, 10% methanol-water, 15% methanol-water solution, and 20% methanol-water solution, collecting fractions, combining fractions with acceptable purity, removing methanol, re-loading the methanol-removed sample onto a C18 reverse-phase chromatographic column, performing gradient elution sequentially with pure water, 10% methanol-water, and 20% methanol-water solution, combining fractions with acceptable purity, and drying, thereby obtaining calcium salvianolate B.

In another preferred embodiment, the magnesium salvianolate B or calcium salvianolate B of the present invention is a salt with high purity obtained by the above-mentioned preparation method, rather than being isolated from natural products.

In a fifth aspect, the present invention provides a use of the pharmaceutical composition according to the first aspect of the present invention or the pharmaceutically acceptable salt of salvianolic acid B according to the second aspect of the present invention in the preparation of a medicament for preventing and/or treating brain diseases.

In another preferred embodiment, the brain disease is cerebrovascular disease or cerebral ischemic disease, preferably, the cerebrovascular disease or cerebral ischemic disease is selected from the group consisting of: ischemic stroke, cerebral arteriosclerosis, cerebral circulation insufficiency, cerebral hemorrhage, cerebral edema, or combinations thereof.

In another preferred embodiment, the brain disease is senile dementia, vascular dementia, brain atrophy, dementia caused by brain atrophy of unknown etiology, as well as neuronal cell loss, behavioral disorders, sensory disorders caused by chronic brain injury, or combinations thereof.

The brain disease is a neurodegenerative disease, preferably, the neurodegenerative disease is selected from the group consisting of: Alzheimer's disease (AD), hippocampal sclerosis, Parkinson's disease, amyotrophic lateral sclerosis, multiple system atrophy, progressive supranuclear palsy, or combinations thereof.

In another preferred embodiment, the preventing and/or treating brain diseases includes improvement of biomarkers of neurodegenerative diseases, the biomarkers selected from the group consisting of: Iba1, neurofilament light chain, GFAP, tau, α-synuclein, or combinations thereof.

In another preferred embodiment, the preventing and/or treating brain diseases is inhibiting white matter atrophy, inhibiting hippocampal atrophy, inhibiting lateral ventricle enlargement, inhibiting striatal atrophy, or combinations thereof.

In another preferred embodiment, the preventing and/or treating brain diseases is inhibiting neuronal cell loss in white matter, inhibiting neuronal cell loss in hippocampus, inhibiting lateral ventricle enlargement, inhibiting neuronal cell loss in striatum, or combinations thereof.

In another preferred embodiment, the preventing and/or treating brain diseases is inhibiting chronic brain tissue necrosis, especially liquefactive necrosis.

In another preferred embodiment, the patient is in the acute, subacute, or chronic phase after the onset of ischemic stroke, such as 0-4.5h, 4.5-6h or 6-24h, e.g., 8h, 12h, 16h, 18h, 20h or even 24h, after the onset of ischemic stroke.

In another preferred embodiment, the pharmaceutical composition is used before, during, or after reperfusion therapy (such as intravenous thrombolysis or mechanical thrombectomy) in patients with cerebral ischemic disease, preferably administered before reperfusion therapy so that the reperfusion therapy can be performed when the
protective effect of the pharmaceutical composition is continuously exerted.

In another preferred embodiment, the patient is a patient who is about to receive, is receiving, or has received reperfusion therapy.

In another preferred embodiment, the patient is a stroke patient who cannot receive reperfusion therapy.

In another preferred embodiment, the pharmaceutical composition of the present invention can be used for preventing or treating other organ injuries caused by ischemic stroke, such as cardiac injury, pulmonary injury, thymic injury, renal injury, or combinations thereof; systemic hypoxia caused by ischemic stroke, or secondary injury due to hypoxia; or reperfusion injury after ischemic stroke, such as tissue and organ damage caused by reperfusion, e.g., cardiac injury, pulmonary injury, thymic injury, renal injury, or combinations thereof.

In a sixth aspect, the present invention provides a method for preventing or treating brain diseases, comprising administering the pharmaceutical composition according to the first aspect of the present invention or the pharmaceutically acceptable salt of salvianolic acid B according to the second aspect of the present invention to a subject in need thereof, thereby preventing or treating the disease.

In another preferred embodiment, the subject is a human or a non-human mammal, such as a rat or mouse.

It should be understood that within the scope of the present invention, the above-mentioned technical features of the present invention and the technical features specifically described below (e.g., in the Examples) can be combined with each other to form new or preferred technical solutions. Due to space limitations, they will not be repeated here.

### Description of Drawings

Figure 1 shows the effect of various salt forms of SalB/Rg1 on reducing infarct volume; compared with Sham group, ***P < 0.001; compared with MCAO group, ###P<0.001; compared with free SalB/Rg1 group, $$P<0.01.
Figure 2 shows that the MLB/Rg1 combination more significantly improves brain tissue structure compared to the free SalB/Rg1 combination.
Figure 3 shows that the MLB/Rg1 combination better maintains neuronal numbers compared to the free SalB/Rg1 combination.
Figure 4 shows that the MLB/Rg1 combination has synergistic effect in the treatment of stroke; Representative TTC staining images of rat brains *(Panel* A) and quantitative results of infarct volume (*Panel* B), as well as evaluation based on the Bliss independent statistical model demonstrate that MLB and Rg1 have a very significant synergistic effect in reducing infarct volume *(Panel* C); compared with Sham, ***P < 0.001; compared with MCAO, #P<0.05, ###P < 0.001; compared with MLB alone group, &&P<0.01; compared with Rg1 alone group, $$$P<0.001.
Figure 5 shows representative images of whole brain coronal sections stained with HE, and representative images of cortex and striatum (scale bar: 50 µm), showing that the MLB/Rg1 combination improves brain tissue structure after stroke.
Figure 6 shows the results of Garcia JH scores on day 1 *(Panel* A), day 3 *(Panel B),* day 5 *(Panel* C), and day 7 *(Panel* D) after stroke; compared with Sham, ***P < 0.001; compared with MCAO group, #P<0.05, ##P<0.01, ###P < 0.001; compared with MLB alone group, &&P<0.01; compared with Rg1 alone group, $P<0.05, $$P<0.01, $$$P<0.001.
Figure 7 shows the results of Longa scores on day 1 (*Panel* A), day 3 (*Panel* B), day *5* (*Panel* C), and day 7 (*Panel* D) after stroke; compared with Sham, ***P < 0.001; compared with MCAO group, #P<0.05, ##P<0.01, ###P < 0.001; compared with MLB alone group, &P<0.05, &&P<0.01; compared with Rg1 alone group, $$P<0.01.
Figure 8 shows representative images of Nissl staining of coronal brain sections of whole brain, cortex and striatum (*Panel* A), and quantitative results of neuronal numbers in cortical and striatal regions (*Panel* B); compared with Sham, ***P < 0.001; compared with MCAO group, #P<0.05, ##P<0.01, ###P < 0.001; compared with MLB alone group, &P<0.05; compared with Rg1 alone group, $P<0.05. (Scale bar: 50 µm).
Figure 9 shows the study protocol for MLB/Rg1 treatment of brain atrophy (*Panel* A) and a general diagram of brain atrophy and TTC staining representative images (*Panel* B).
Figure 10 shows quantitative results of brain atrophy; compared with Sham group *p<0.05, **p<0.01, ***p<0.001; compared with MCAO #p<0.05, ##p<0.01, ###p<0.001; compared with Rg1 &p<0.05, &&p<0.01, &&&p<0.001; compared with MLB $p<0.05, $$p<0.01, $$$p<0.001; ^ represents compared with 5 (1:10) mg/kg MLB/Rg1 p<0.05, ^^p<0.01, ^^^p<0.001.
Figure 11 shows the statistical results of the beam walking test; compared with Sham group *p<0.05, **p<0.01, ***p<0.001; compared with MCAO #p<0.05, ##p<0.01, ###p<0.001.
Figure 12 shows representative images of HE staining.
Figure 13 shows representative images of Nissl staining.
Figure 14 shows quantitative results of Nissl staining; compared with Sham group *p<0.05, **p<0.01, ***p<0.001; compared with MCAO #p<0.05, ##p<0.01, ###p<0.001; compared with Rg1 &p<0.05, ^{&&}p<0.01, ^{&&&}p<0.001; compared with MLB ^{$}p<0.05, ^{$$}p<0.01, ^{$$$}p<0.001; compared with 5(1:10) mg/kg MLB/Rg1 ^p<0.05, ^^p<0.01, ^^^p<0.001.
Figure 15 shows the evaluation of the synergistic effect of MLB and Rg1 in reducing infarct volume (*Panel* A) and inhibiting brain atrophy (*Panel* B) using the Bliss method.
Figure 16 shows overall TTC staining images of brain.
Figure 17 shows quantitative results of brain atrophy. Compared with Sham group *p<0.05, **p<0.01, ***p<0.001; compared with MCAO #p<0.05, ##p<0.01, ###p<0.001;
Figure 18 shows representative images of HE staining.
Figure 19 shows representative images of Nissl staining.
Figure 20 shows quantitative results of Nissl staining; compared with Sham group *p<0.05, **p<0.01, ***p<0.001; compared with MCAO #p<0.05, ##p<0.01, ###p<0.001.
Figure 21 shows the evaluation of the down-regulation effects of different ratios of MLB/Rg1 on infarct volume using TTC staining.
Figure 22 shows the evaluation of the down-regulation effect of SalA magnesium salt/Rb1 on infarct volume (*Panel* A) and effects thereof in improving neurological behavior (*Panel* B, *Panel* C) using TTC staining.
Figure 23 shows the evaluation of the down-regulation effect of SalA magnesium salt/Rh2 group on infarct volume (*Panel* A) and effects thereof in improving neurological behavior (*Panel* B, *Panel* C) using TTC staining.
Figure 24 shows the evaluation of the down-regulation effects of RosA-Na/Rb1 with different ratios on infarct volume (*Panel* A), and the neurological behavior evaluation BWT Score (*Panel* B) and Garcia JH score (*Panel* C) for RosA-Na/Rb1 groups with different ratios.
Figure 25 shows the evaluation of the down-regulation effect of MLB/Rg1 via sublingual administration on infarct volume (*Panel* A- *Panel* B), BWT evaluation *(Panel* C), and Garcia JH score (*Panel* D) using TTC staining.
Figure 26 shows survival curves of different groups, compared with Sham group, **P < 0.01; compared with tMCAO group, #P < 0.05.
Figure 27 shows that MLB/Rg1 treatment reduced brain edema in tMCAO mice.
Figure 28 shows that MLB/Rg1 improves neurological behavior in mice by protecting brain neurons.
Figure 29 shows magnetic resonance images of mouse cerebral blood flow (CBF).
Figure 30 shows that MLB/Rg1 significantly improves cerebral blood flow in the infarct border zone.
Figure 31 shows that administration of MLB/Rg1 at 24 hours after infarction still reduces infarct volume, representative images of TTC staining (*Panel* A), quantitative results of infarct volume (*Panel* B).
Figure 32 shows that administration of MLB/Rg1 at 24 hours after infarction reduces infarct volume and has a synergistic effect, as shown in the Bliss non-independent model statistics.
Figure 33 shows that, in the evaluation of tissue sampled seven days later, the administration of MLB/Rg1 at 24 hours after infarction, prolongs coagulation time (*Panel* A) and improves neurological behavior (*Panel* B).
Figure 34 shows that, in evaluation of tissue sampled seven days later, the administration of MLB/Rg1 at 24 hours after infarction improves blood oxygen content (*Panel* A) and enhances cardiac systolic function (*Panel* B).
Figure 35 shows that, in evaluation of tissue sampled seven days later, the administration of MLB/Rg1 at 24 hours after infarction reduces thymic (*Panel* A) and pulmonary (*Panel* B) injury after stroke.

### Detailed Description of the Invention

The present inventors, after extensive and in-depth research and through extensive screening and testing, provide a medicament with significantly improved prevention or treatment of brain diseases. The present invention unexpectedly discovered for the first time that, compared with the combination of free salvianolic acid B (SalB) and ginsenoside Rg1, the combination of a specific salt of salvianolic acid B and ginsenoside Rg1 has significantly improved therapeutic effects in acute, subacute, and chronic brain injury or neurodegenerative diseases, thus completing the present invention.

### Terms

Unless otherwise defined, all technical and scientific terms used herein have the same meaning as commonly understood by one of ordinary skill in the art to which this invention belongs.

As used herein, the term "comprising" or "including" may be open-ended, semi-closed, or closed-ended. In other words, the term also includes "consisting essentially of" or "consisting of".

As used herein, the term "room temperature" or "ambient temperature" refers to a temperature of 4-40 °C, preferably 25±5 °C.

### Active Ingredients

In the present invention, the first active ingredient is a pharmaceutically acceptable salt of salvianolic acid, preferably a pharmaceutically acceptable salt of salvianolic acid B. As used herein, "pharmaceutically acceptable salt of salvianolic acid" refers to a salt that is suitable for use as a medicament of salvianolic acid formed with an acid or base. Pharmaceutically acceptable salts include inorganic and organic salts. One preferred class of salts are salts formed by the compounds of the active ingredients of the present invention with acids. Acids suitable for salt formation include, but are not limited to: inorganic acids such as hydrochloric acid, hydrobromic acid, hydrofluoric acid, sulfuric acid, nitric acid, phosphoric acid; organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, toluenesulfonic acid, and benzenesulfonic acid; and acidic amino acids such as aspartic acid and glutamic acid. One preferred class of salts are salts formed by the compounds of the active ingredients of the present invention with bases. Bases suitable for salt formation include, but are not limited to: inorganic bases such as sodium hydroxide, potassium hydroxide, sodium carbonate, sodium bicarbonate, sodium phosphate, calcium carbonate, magnesium carbonate; organic bases such as ammonia water, trimethylamine, triethylamine, diethylamine, diethanolamine, piperazine. Another preferred class of salts are salts formed by the active ingredients of the present invention with metal ions, including but not limited to (mono)magnesium salts, (mono)calcium salts, (di)sodium salts, (di)potassium salts, (di)lithium salts, zinc salts, and (di)silver salts, etc., and salts formed with (natural) amino acids such as arginine, lysine, etc.

Preferably, the salt is a magnesium salt of salvianolic acid B.

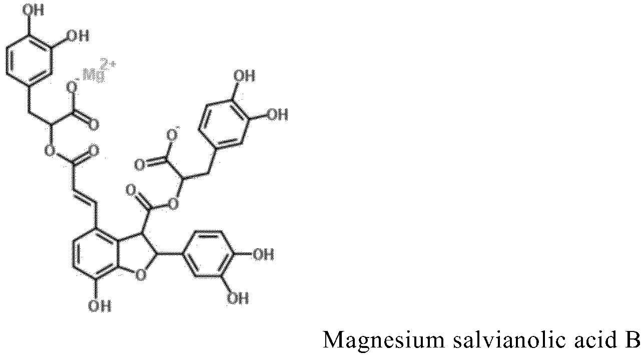

In another preferred embodiment, the purity of magnesium salvianolate B in the first active ingredient is ≥ 98%, preferably ≥ 98.5%, more preferably ≥ 99% or 99.5%, by weight.

In the present invention, the second active ingredient is ginsenoside, or a pharmaceutically acceptable salt thereof, preferably ginsenoside Rg1, or a pharmaceutically acceptable salt thereof.

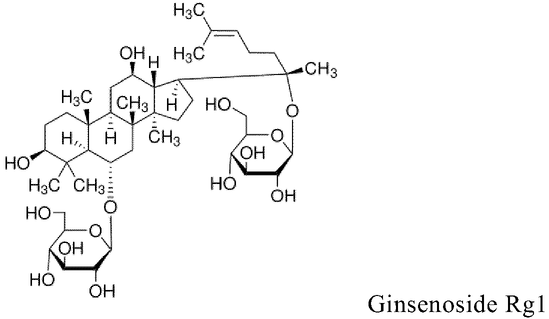

In the present invention, the "pharmaceutically acceptable salt" suitable for ginsenoside includes salts of ginsenoside that are suitable for use as a medicament formed with an acid or base. Pharmaceutically acceptable salts include inorganic and organic salts. One preferred class of salts are salts formed by the compounds of the active ingredients of the present invention with acids. Acids suitable for salt formation include, but are not limited to: inorganic acids such as hydrochloric acid, hydrobromic acid, hydrofluoric acid, sulfuric acid, nitric acid, phosphoric acid; organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, toluenesulfonic acid, benzenesulfonic acid; and acidic amino acids such as aspartic acid and glutamic acid. One preferred class of salts are salts formed by the compounds of the active ingredients of the present invention with bases. Bases suitable for salt formation include, but are not limited to: inorganic bases such as sodium hydroxide, potassium hydroxide, sodium carbonate, sodium bicarbonate, sodium phosphate; organic bases such as ammonia water, trimethylamine, triethylamine, diethylamine, diethanolamine, piperazine. Another preferred class of salts are salts formed by the active ingredients of the present invention with metal ions, including but not limited to magnesium salts, sodium salts, calcium salts, potassium salts, lithium salts, zinc salts, and silver salts, etc., and salts with (natural) amino acids such as arginine, lysine, etc.

Preferably, apart from unavoidable impurities, the active ingredients of the present invention consist only of the aforementioned first active ingredient and second active ingredient.

Preferably, the weight ratio of the first active ingredient to the second active ingredient is 1:(0.1-10), wherein the weight ratio is calculated based on magnesium salvianolate B and ginsenoside Rg1 (the parent compound).

In another preferred embodiment, the weight ratio of the first active ingredient to the second active ingredient is 1:(0.2-5), preferably 1:(0.4-0.6) or 1:(2-3).

In another preferred embodiment, the weight ratio of the first active ingredient to the second active ingredient is 1:0.1, 1:0.15, 1:0.2, 1:0.25, 1:0.3, 1:0.35, 1:0.4, 1:0.45, 1:0.5, 1:0.55, 1:0.6, 1:0.65, 1:0.7, 1:0.75, 1:0.8, 1:0.85, 1:0.9, 1:0.95, 1:1, 1:1.2, 1:1.4, 1:1.5, 1:1.6, 1:1.8, 1:2, 1:2.2, 1:2.5, 1:2.8, 1:3, 1:3.2, 1:3.5, 1:4, 1:4.5, 1:5, 1:5.5, 1:6, 1:6.5, 1:7, 1:7.5, 1:8, 1:8.5, 1:9, 1:9.5 or 1:10.

Preferably, the present invention provides a preferred pharmaceutically acceptable salt of salvianolic acid B, wherein the salt is magnesium salvianolate B or calcium salvianolate B.

In another preferred embodiment, the purity of magnesium salvianolate B is ≥ 80%, preferably ≥ 90%, more preferably ≥ 95%, 98%, ≥ 99% or 99.5%.

In another preferred embodiment, the purity of calcium salvianolate B is ≥ 80%, preferably ≥ 90%, more preferably ≥ 95%, 98%, ≥ 99% or 99.5%.

In the present application, when the magnesium salt or calcium salt of salvianolic acid B is used as an active ingredient in the pharmaceutical composition, the purity thereof refers to a purity thereof in the separable individual active pharmaceutical ingredient.

In another preferred embodiment, the pharmaceutical composition further comprises (c) a pharmaceutically acceptable carrier.

### Blood-Brain Barrier

The blood-brain barrier refers to barriers between plasma and brain formed by the brain capillary wall and glial cells, and barriers between plasma and cerebrospinal fluid formed by the choroid plexus. These barriers are able to prevent certain substances from entering brain tissues from the blood.

The existence of the blood-brain barrier is of great significance for preventing harmful substances from entering the brain, but it also prevents the transfer of most small molecule and macromolecule drugs (e.g., peptides, proteins, and nucleic acids), severely limiting the treatment of central nervous system diseases (e.g., neurodegenerative diseases, brain tumors, brain infections, and stroke).

In most cases, penetration of the blood-brain barrier depends on the lipophilicity of the compound, which is defined as the equilibrium partition coefficient of the non-ionized compound between two immiscible phases (e.g., n-octanol and water/buffer), and is one of the most important physicochemical properties of a compound. Lipophilicity affects the absorption, distribution, metabolism, excretion, toxicity, and pharmacological activity of the compound, and is also a parameter closely related to blood-brain barrier penetration. By improving lipophilicity, the blood-brain barrier penetration of drugs can be enhanced.

However, the present application unexpectedly found that after using a salt of salvianolic acid B (especially the magnesium salt thereof) in combination with Rg1 (after salt formation, hydrophilicity is enhanced, and lipophilicity is reduced), compared with the combination of free salvianolic acid B and Rg1, the brain protection and therapeutic effect on brain diseases are significantly improved, not only in cerebrovascular diseases such as stroke, but also unexpectedly found that the salt of salvianolic acid B/Rgl combination has a significantly improved and unexpected therapeutic effect on degenerative diseases represented by Alzheimer's disease and vascular dementia, which is extremely surprising to those skilled in the art.

In addition, the present application also found that the combination of magnesium salvianolate B and Rg1 has significantly higher bioavailability and efficacy when administered sublingually/orally/mucosally, making it highly suitable for the preparation of emergency drugs for brain diseases administered sublingually.

### Pharmaceutical Composition and Uses

The present invention provides a pharmaceutical composition, wherein the active ingredients of the pharmaceutical composition may comprise (a) and (b):
(a) a first active ingredient, which is a pharmaceutically acceptable salt of salvianolic acid; and (b) a second active ingredient, which is ginsenoside, or a pharmaceutically acceptable salt thereof; and (c) a pharmaceutically acceptable carrier.

Typically, salvianolic acids useful in the present invention include, but are not limited to: salvianolic acid B, salvianolic acid A, salvianolic acid C, lithospermic acid, rosmarinic acid, tanshinol, protocatechualdehyde, yunnaneic acid A, ferulic acid, salvianolic acid D, salvianolic acid E, salvianolic acid F, salvianolic acid G, salvianolic acid L, caffeic acid, isoferulic acid, methyl rosmarinate, salviaflaside, lithospermic acid, salvinal, dimethyl lithospermate, etc., preferably salvianolic acid B or salvianolic acid A.

Typically, ginsenosides useful in the present invention include, but are not limited to: ginsenoside Ra3, Rb1, Rb2, Rb3, Rc, Rd, Rg3, F, CK, Mc, Rg3, Rh2, Re, Rg1, Rg2, Rh1, F1, R1; notoginsenoside R1, R2, R3, R6, M, N, Rt, T3, FP1, N, Rw1, etc., preferably ginsenoside Rg1 or ginsenoside Rb1.

Preferably, the pharmaceutical composition of the present invention comprises: (a) a first active ingredient, which is a pharmaceutically acceptable salt of salvianolic acid B; and (b) a second active ingredient, which is ginsenoside Rg1, or a pharmaceutically acceptable salt thereof; and (c) a pharmaceutically acceptable carrier; wherein the pharmaceutically acceptable salt of salvianolic acid B is selected from the group consisting of: magnesium salvianolate B, calcium salvianolate B, or combinations thereof. More preferably, the first active ingredient is magnesium salvianolate B; and the second active ingredient is ginsenoside Rg1.

Preferably, the pharmaceutical composition of the present invention comprises: (a) a first active ingredient, which is a pharmaceutically acceptable salt of salvianolic acid A; and (b) a second active ingredient, which is ginsenoside Rh2, or a pharmaceutically acceptable salt thereof; and (c) a pharmaceutically acceptable carrier.

Preferably, the pharmaceutical composition of the present invention comprises: (a) a first active ingredient, which is a pharmaceutically acceptable salt of salvianolic acid A; and (b) a second active ingredient, which is ginsenoside Rb1, or a pharmaceutically acceptable salt thereof; and (c) a pharmaceutically acceptable carrier.

Preferably, the pharmaceutical composition of the present invention comprises: (a) a first active ingredient, which is a pharmaceutically acceptable salt of rosmarinic acid; and (b) a second active ingredient, which is ginsenoside Rb1, or a pharmaceutically acceptable salt thereof; and (c) a pharmaceutically acceptable carrier.

The present application unexpectedly found that the salt of salvianolic acid B (especially the magnesium salt thereof), compared with free salvianolic acid B, after combining with Rg1, significantly improves the brain protection and therapeutic effect on brain diseases, not only in cerebrovascular diseases such as stroke, but also unexpectedly found that the salt of salvianolic acid B/Rgl combination has a significantly improved and unexpected therapeutic effect on degenerative diseases represented by Alzheimer's disease and vascular dementia.

The "preventing" and "treating" of the present invention include delaying and halting the progression of the disease, or eliminating the disease, and do not require 100% inhibition, elimination, or reversal. In some embodiments, compared with the levels observed in the absence of the composition or pharmaceutical composition of the present invention, the composition or pharmaceutical composition of the present invention prevents, alleviates, inhibits, and/or reverses ischemia-reperfusion injury by, for example, at least about 10%, at least about 30%, at least about 50%, or at least about 80%.

In the pharmaceutical composition of the present invention, the first active ingredient and the second active ingredient can be formulated separately or mixed together to prepare a formulation.

The pharmaceutical composition of the present invention contains a safe and effective amount of the first active ingredient and/or the second active ingredient. The "safe and effective amount" means an amount of the active ingredient that is sufficient to significantly improve the condition without causing serious side effects. Typically, the pharmaceutical composition contains 1-2000 mg of the active ingredient of the present invention per dose, more preferably, 10-500 mg of the active ingredient of the present invention per dose. Preferably, "one dose" is a capsule, a tablet, an injection, etc.

In the present invention, "pharmaceutically acceptable carrier" refers to: one or more compatible solid or liquid fillers or gel substances, which are suitable for human use and must be of sufficient purity and sufficiently low toxicity. "Compatibility" herein means that the components of the composition can be mixed with the first active ingredient and/or the second active ingredient without significantly reducing the efficacy of the first active ingredient and/or the second active ingredient. Examples of pharmaceutically acceptable carriers include cellulose and its derivatives (e.g., sodium carboxymethylcellulose, sodium ethylcellulose, cellulose acetate, etc.), gelatin, talc, solid lubricants (e.g., stearic acid, magnesium stearate), calcium sulfate, vegetable oils (e.g., soybean oil, sesame oil, peanut oil, olive oil, etc.), polyols (e.g., propylene glycol, glycerin, mannitol, sorbitol, etc.), emulsifiers (e.g., Tween^{®}), wetting agents (e.g., sodium lauryl sulfate), colorants, flavoring agents, stabilizers, antioxidants, preservatives, pyrogen-free water, etc.

Solid dosage forms such as tablets, dragees, capsules, pills, and granules can be prepared with coatings and shell materials, such as enteric coatings and other materials well known in the art. They may contain opacifying agents, and the release of the active ingredients in such compositions may be delayed and released in a certain part of the digestive tract. Examples of embedding components that can be used are polymeric substances and waxes. If necessary, the active ingredients can also be microencapsulated with one or more of the above excipients.

Liquid dosage forms for oral administration include pharmaceutically acceptable emulsions, solutions, suspensions, syrups, or tinctures. In addition to the active ingredients, the liquid dosage forms may contain inert diluents conventionally used in the art, such as water or other solvents, solubilizers, and emulsifiers, for example, ethanol, isopropanol, ethyl carbonate, ethyl acetate, propylene glycol, 1,3-butylene glycol, dimethylformamide, and oils, especially cottonseed oil, peanut oil, corn germ oil, olive oil, castor oil, and sesame oil, or mixtures of these substances.

In addition to these inert diluents, the composition may also contain adjuvants, such as wetting agents, emulsifying agents, and suspending agents, sweetening agents, flavoring agents, and perfuming agents.

In addition to the active ingredients, suspensions may contain suspending agents, for example, ethoxylated isostearyl alcohol, polyoxyethylene sorbitol and sorbitan esters, microcrystalline cellulose, aluminum methoxide, and agar, or mixtures of these substances.

A preferred dosage form is a preparation for oral administration, such as a sublingual or buccal mucosal dosage form, e.g., orally disintegrating tablet, mucoadhesive tablet, sublingual tablet, buccal tablet, chewable tablet, effervescent tablet, solution, etc. In addition, those skilled in the art can make various modifications to the tablets as needed. It can also be an intraorally dispersible preparation, such as an oral dissolving film, orally disintegrating tablet, suspension, suspension tablet, rapid disintegrating tablet, orally disintegrating granule, orally disintegrating lozenge, sublingual tablet, powder, and/or chewable tablet. When considering the administration situation, portability, and various purposes of the pharmaceutical preparation, the pharmaceutical preparation according to the present disclosure can be prepared as a suspension preparation, oral dissolving film preparation, rapid disintegrating tablet, or orally disintegrating granule. In particular, 80% or more of the preparation can dissolve, disperse, or disintegrate within 10 minutes or 5 minutes after oral administration.

The term oral dissolving film refers to a preparation that adheres to the oral cavity, for example, on the tongue, oral mucosa, sublingually, etc. When the pharmaceutical preparation for oral administration according to the present disclosure is prepared as an oral dissolving film, it may contain a polymer to form a film, such as pullulan, hydroxypropyl cellulose (HPC), hydroxypropyl methylcellulose (HPMC), polyvinylpyrrolidone (PVP), starch, polyethylene glycol-polyvinyl alcohol copolymer, copovidone, hydroxyethyl cellulose, hydroxypropyl starch, polyethylene oxide, poloxamer, or mixtures thereof. Based on the weight of the dry film, the content of the polymer may be 20%-50% by weight, but is not limited thereto.

Preferably, the preparation for oral administration contains a taste-masking agent, such as β-cyclodextrin, α-cyclodextrin, and γ-cyclodextrin, or their derivatives containing hydroxypropyl or methyl groups. Cyclodextrins can not only mask bitterness but also help in the uniform dispersion of the active ingredients.

Compositions for parenteral injection may include physiologically acceptable sterile aqueous or non-aqueous solutions, dispersions, suspensions, or emulsions, and sterile powders for reconstitution into sterile injectable solutions or dispersions. Suitable aqueous and non-aqueous carriers, diluents, solvents, or excipients include water, ethanol, polyols, and suitable mixtures thereof.

Dosage forms for topical administration of the active ingredients of the present invention include ointments, powders, patches, sprays, and inhalants. The active ingredients are mixed under sterile conditions with a physiologically acceptable carrier and any preservatives, buffers, or propellants that may be required.

In the pharmaceutical composition, combination of active ingredients, or kit of the present invention, the generally effective therapeutic dose range of the active ingredients, based on the total amount of the salt of salvianolic acid B and ginsenoside Rg1, will be: about 1-2000 mg/day, about 10-about 1000 mg/day, about 10-about 500 mg/day, about 10-about 250 mg/day, about 10-about 100 mg/day, or about 10-about 80 mg/day. The therapeutically effective dose will be administered in one or more doses. However, it should be understood that the specific dose of the active ingredient of the present invention for any particular patient will depend on various factors, such as the age, sex, weight, general health, diet, individual response, time of administration, severity of the disease to be treated, dosage form, mode of application, and concomitant medications. The therapeutically effective amount for a given situation can be determined by routine experimentation and is within the capability and judgment of the clinician or physician. In any case, the active ingredient will be administered in multiple doses based on the individual circumstances of the patient and in a manner that allows delivery of a therapeutically effective amount.

### Preparation Methods

The present invention provides a method for preparing a pharmaceutically acceptable salt of salvianolic acid, comprising a step of: in water or an aqueous solvent, reacting free salvianolic acid with a substance containing the desired cation, thereby generating the pharmaceutically acceptable salt of salvianolic acid.

Preferably, the present invention provides a method for preparing a pharmaceutically acceptable salt of salvianolic acid B, comprising a step of: in water or an aqueous solvent, reacting free salvianolic acid B with a substance containing the desired cation, thereby generating the pharmaceutically acceptable salt of salvianolic acid B.

More preferably, the present invention provides a method for preparing magnesium salvianolate B, comprising a step of: in water or an aqueous solvent, reacting free salvianolic acid B with an inorganic salt or inorganic base containing magnesium ions, thereby generating magnesium salvianolate B.

In another preferred embodiment, the inorganic salt or inorganic base containing magnesium ions is selected from the group consisting of: magnesium carbonate, magnesium chloride, magnesium sulfate, magnesium nitrate, magnesium hydroxide, magnesium bicarbonate, or combinations thereof.

In another preferred embodiment, the method comprises steps of: adding an aqueous solution of free salvianolic acid B to an aqueous solution of an inorganic salt or inorganic base containing magnesium ions, thereby reacting to generate magnesium salvianolate B.

In another preferred embodiment, the molar ratio of free salvianolic acid to the inorganic salt or inorganic base containing magnesium ions is 1:0.98-1.02, preferably 1:0.99-1.01, more preferably 1:1.

In another preferred embodiment, the method further comprises steps of: loading the above reaction solution onto a C18 reverse-phase chromatographic column, performing gradient elution sequentially with pure water, 10% methanol-water, 15% methanol-water solution, and 20% methanol-water solution, collecting fractions, combining fractions with acceptable purity, removing methanol, re-loading the methanol-removed sample onto a C18 reverse-phase chromatographic column, performing gradient elution sequentially with pure water, 10% methanol-water, and 20% methanol-water solution, combining fractions with acceptable purity, and drying, thereby obtaining magnesium salvianolate B.

The present invention also provides a method for preparing calcium salvianolate B, comprising a step of: in water or an aqueous solvent, reacting free salvianolic acid B with an inorganic salt or inorganic base containing calcium ions, thereby generating calcium salvianolate B.

In another preferred embodiment, the inorganic salt or inorganic base containing calcium ions is selected from the group consisting of: calcium carbonate, calcium sulfate, calcium nitrate, calcium hydroxide, or combinations thereof.

In another preferred embodiment, the method comprises steps of: adding an aqueous solution of free salvianolic acid B to an aqueous solution of an inorganic salt or inorganic base containing calcium ions, thereby reacting to generate calcium salvianolate B.

In another preferred embodiment, the molar ratio of free salvianolic acid to the inorganic salt or inorganic base containing calcium ions is 1:0.98-1.02, preferably 1:0.99-1.01, more preferably 1:1.

In another preferred embodiment, the method further comprises steps of: loading the above reaction solution onto a C18 reverse-phase chromatographic column, performing gradient elution sequentially with pure water, 10% methanol-water, 15% methanol-water solution, and 20% methanol-water solution, collecting fractions, combining fractions with acceptable purity, removing methanol, re-loading the methanol-removed sample onto a C18 reverse-phase chromatographic column, performing gradient elution sequentially with pure water, 10% methanol-water, and 20% methanol-water solution, combining fractions with acceptable purity, and drying, thereby obtaining calcium salvianolate B.

Typically, the purity in the "acceptable purity" refers to a purity standard defined according to needs, which can be selected according to actual needs, such as a purity of ≥ 80%, preferably ≥ 90%, more preferably ≥ 95%, 98%, ≥ 99%, or 99.5%.

Furthermore, the present invention also provides a method for preparing the pharmaceutical composition of the present invention, comprising steps of:
Mixing the first active ingredient, the second active ingredient, and the pharmaceutically acceptable carrier uniformly in any order, thereby obtaining the pharmaceutical composition.

**The main advantages of the present invention include:**
The present invention unexpectedly discovered for the first time that, compared with the combination of free salvianolic acid B and ginsenoside Rg1, the combination of a specific salt of salvianolic acid B and ginsenoside Rg1 has significantly improved synergistic effects and therapeutic effects in preventing or treating brain diseases, thus providing a drug with better efficacy.

The present invention will be further elaborated below in conjunction with specific examples. It should be understood that these examples are only for illustrating the present invention and are not intended to limit the scope of the present invention. Experimental methods without specific conditions indicated in the following examples are generally performed according to conventional conditions or according to conditions recommended by the manufacturer. Unless otherwise indicated, percentages and parts are calculated by weight.

**Table 1: Abbreviations**

| Abbreviation | Meaning |
|---|---|
| SalB | Free salvianolic acid B |
| SalB-Ca | Calcium salvianolate B |
| MLB | Magnesium salvianolate B |
| SalB/Rg1 | Combination of free salvianolic acid B and ginsenoside Rg1 |
| SalB-Ca/Rg1 | Combination of calcium salvianolate B and ginsenoside Rg1 |
| MLB/Rg1 | Combination of magnesium salvianolate B and ginsenoside Rg1 |

### General Methods

### I. Experimental Materials and Instruments

1. Materials: Salvia miltiorrhiza tablets, methanol (analytical grade), acetonitrile (analytical grade), phosphoric acid (analytical grade). Column chromatography silica gel. Magnesium carbonate. Chromatographic grade acetonitrile. Clippers; surgical scissors, small curved forceps, ophthalmic scissors, and microvascular clips; suture needles and threads; medical absorbent cotton and cotton swabs (Sinopharm Chemical Reagent Co., Ltd.); syringes; filament sutures (Shanghai Yuyan Scientific Instrument Co., Ltd., model: M8510); aluminum foil; 6-well cell culture plates (NUNC); microporous membranes (Millex-GP Filter Unit). Zoletil 50 (Virbac, France); iodophor (Shanghai Likang Disinfection High-tech Co., Ltd.); 2,3,5-triphenyltetrazolium chloride (Sigma); sodium chloride injection (Heilongjiang Qitao Animal Health Products Co., Ltd.); 75% ethanol (Sinopharm Chemical Reagent Co., Ltd.). Storage conditions for salvianolic acid B, magnesium salvianolate B (MLB), calcium salvianolate B (SalB-Ca), and ginsenoside Rg1 were all -20°C, protected from light.
2. Instruments: Medium-pressure C18 reverse-phase chromatographic column, high-pressure C18 reverse-phase chromatographic column, HPLC analytical column, medium-pressure preparative instrument, analytical high-performance liquid chromatograph, preparative high-performance liquid chromatograph, ultrasonic cleaner, rotary evaporator, vacuum drying oven, freeze dryer, low-temperature refrigerator, vacuum pump, magnetic stirrer, cold light source, animal constant temperature system, electronic balance, electronic balance, vertical pressure steam sterilizer, centrifuge, vortex mixer, ultra-low temperature refrigerator, stereo microscope, electric blast drying oven.
3.Experimental Animals: SD rats and C57BL/6 mice were housed in the SPF animal facility of the Shanghai Institute of Materia Medica, with free access to food and water, a 12-hour light/12-hour dark cycle, and a temperature controlled at 22 ± 2°C. Animals were acclimatized for three days before experiments. All animal care and experimental procedures were approved by the Institutional Animal Care and Use Committee of the Shanghai Institute of Materia Medica, and the experiments were strictly conducted in accordance with the "Guide for the Care and Use of Laboratory Animals".

### II. General Methods for Model Preparation and Detection

### 1. Establishment of Permanent Cerebral Ischemia Animal Model

### 1.1 Preoperative Preparation

Surgical instruments, cotton swabs, absorbent cotton, and other surgical supplies were sterilized in an autoclave at 120°C for 30 min before surgery and set aside; test substance solutions needed for the day were taken out from a -20°C freezer and allowed to return to room temperature; all experimental animals were weighed and subjected to behavioral tests before surgery; the surgical area was cleaned and disinfected, and surgical supplies were prepared.

### 1.2 Surgical Anesthesia

Rats or mice were anesthetized with Zoletil 50 via intraperitoneal injection. When the anesthesia depth was sufficient that the animal could not freely turn over, the neck was shaved using a clipper and depilatory cream, and the animal was fixed on a sterilized surgical board. The neck was disinfected again with iodophor.

### 1.3 Establishment of Permanent Cerebral Ischemia Animal Model via Middle Cerebral Artery Occlusion

The skin of the animal's neck was incised along the midline with ophthalmic scissors, subcutaneous tissues and fat were separated, exposing the deep cervical fascia and anterior tracheal muscles, care was taken not to damage the nerves during vessel separation. After isolating the carotid artery, a 5-0 silk suture of appropriate length was passed around the proximal end to block blood flow in the left common carotid artery. A small opening was cut in the proximal end of the common carotid artery with micro-scissors, and a silicone-coated filament suture was gently inserted. Whether the filament suture reached the bifurcation of the anterior cerebral artery and middle cerebral artery was determined based on the insertion depth and resistance. The filament suture was fixed with a slipknot tied with the silk suture at distal common carotid artery. According to the experimental design, tissue sampled at 24 h after middle cerebral artery occlusion was used to evaluate the protective effect in the acute phase of infarction; tissue sampled at 7 days was used to evaluate the subacute phase effect; and tissue sampled at 28 days was used to evaluate the chronic phase of infarction, as well as the therapeutic effects on brain atrophy, vascular dementia, Alzheimer's disease, etc.

### 2. Establishment of Reperfusion Cerebral Ischemia Animal Model

Zoletil 50 was administered intraperitoneally. After anesthesia, the neck to the mandible area was shaved with a clipper, the animal was tied to a board with a rope, and the skin on the right side of the neck was disinfected with an iodophor cotton ball and incised. The subcutaneous connective tissue between the bilateral bulging glands was separated along the midline, and the anterior cervical muscles between the right muscle masses were separated.Vessels and nerves around common carotid artery (CCA) were carefully dissected to isolate the CCA. The CCA bifurcation was temporarily clamped with a microvascular clip, the proximal end of the CCA was tightly ligated with a suture, and a thread was left around the distal end. A small oblique incision was made in the upper part of the CCA between the two ligatures using ophthalmic scissors. The filament suture was inserted through the small opening in the CCA using ophthalmic forceps. After insertion, the filament suture was lightly tied with the thread around the distal CCA. The filament suture was gently pushed with ophthalmic forceps, and the distance was measured from the vessel bifurcation. Whether the filament suture reached the middle cerebral artery (MCA) was determined based on the insertion depth and resistance. After cutting the excess suture outside the vessel, 1 ml of 4X antibiotic solution was dripped onto the wound to prevent infection, the wound was then sutured, and the animal was placed on a heating pad. After the specified ischemic time (3h, 6h, 9h, 12h, or 24h), the wound sutures were removed, and the filament suture was pulled out to achieve reperfusion. 1 ml of 4X antibiotic solution was dripped onto the wound to prevent infection, the wound was then sutured, and the animal was placed on a heating pad until it regained consciousness, then transferred to the breeding room. According to the experimental design, sample taken at 24 h or 72 h after reperfusion was used to evaluate the protective effect in the acute phase of infarction; sample taken at 7 days after reperfusion was used to evaluate the subacute phase effect; and sample taken at 28 days after reperfusion was used to evaluate the chronic phase of infarction, as well as the therapeutic effects on brain atrophy, vascular dementia, Alzheimer's disease, etc.

### 3. Behavioral Evaluation

Behavioral evaluation is one of the important indicators for clinicians to assess the severity of ischemic stroke patients, which is performed based on the patient's behavioral abilities to determine the degree of neurological function impairment. According to the experimental design, the researchers evaluated animal behavior using Longa score, Garcia JH score, etc., on day1, 3, 5, 7, 14, and 28 after the establishment of the permanent cerebral ischemia model or the reperfusion cerebral ischemia model.

### 3.1 Longa Score

The Longa score is a classic method for behavioral evaluation in animals both domestically and internationally, providing a comprehensive assessment of the animal's motor behavior. Taking left-sided cerebral ischemia as an example, the scoring criteria are as follows: 0 point represents normal limbs, indicating no neurological behavioral deficits; 1 point represents incomplete extension of the right limb, indicating mild neurological behavioral deficits; 2 points represent circling to the right when walking, indicating moderate neurological behavioral deficits; 3 points represent falling to the right when walking, indicating severe neurological behavioral deficits; 4 points represent inability to walk spontaneously, indicating loss of consciousness. That is, a higher Longa score indicates more severe neurological behavioral deficits in the rat.

### 3.2 Garcia JH Score

The Garcia JH score is also one of the commonly used methods for behavioral evaluation in animals. After the onset of ischemic stroke, patients' motor, sensory, visual, and language functions may be significantly affected. The recovery of neurological function after stroke is not fully understood, and the recovery of neurological function in stroke patients may not only be related to the size of the cerebral infarct volume but also to the location and degree of the deficit. Therefore, the motor and sensory functions of animals were separately assessed using the Garcia JH score. The scoring criteria of the Garcia JH score are shown in Table 2. The first 4 items evaluate the animal's motor function, and the last 2 items evaluate the animal's sensory function. The evaluation result ranges from a minimum of 3 points to a maximum of 18 points, with higher scores indicating better neurological function. 3-7 points represent severe neurological dysfunction, 8-12 points represent moderate neurological dysfunction, and 13-16 points represent mild neurological dysfunction. The "Sensory score" herein is a comprehensive evaluation of the bilateral body touch reflex and bilateral vibrissae touch reflex in the following table.

**Table 2: Garcia JH Scoring Criteria**

| Test | 0 | 1 | 2 | 3 |
|---|---|---|---|---|
| Spontaneous activity (Place animal in cage, observe for 5 min) | No spontaneous movement | Minimal movement | Movement and touching at least one side of cage | Movement and touching at least three sides of cage |
| Symmetry of posture (Suspend by tail, observe limb state) | No movement on affected side | Slight movement on affected side | Slower movement on affected side | Bilateral symmetry |
| Forelimb extension (Suspend by tail so hind limbs are suspended, forelimbs move towards table, observe forelimb extension) | No extension on affected side | Slight extension on affected side | Extension on affected side but less than normal side | Bilateral symmetrical extension |
| Grid test | --- | Cannot climb | Affected side weaker | Normal climbing |
| Bilateral body touch reflex | --- | No response on affected side | Weaker response on affected side than normal side | Same reactivity |
| Bilateral vibrissae touch reflex | --- | No response on affected side | Weaker response on affected side than normal side | Same reactivity |

### 3.3 Beam Walking Test (BWT)

The beam walking test can evaluate the integration and coordination of the animal's motor function. A wooden plank is placed horizontally, and animals are individually placed on it, allowing the test animal to remain upright and cross a narrow elevated beam to a safe platform. The BWT score ranges from 1 to 7: 7 points, successfully traverses the beam, paralyzed limb is fully functional, no obvious neurological deficits; 6 points, traverses the beam, paralyzed limb is >50% functional; 5 points, traverses the beam, paralyzed limb is <50% functional; 4 points, cannot smoothly traverse the beam, fall rate <50%; 3 points, cannot smoothly traverse the beam, fall rate >50%; 2 points, cannot crawl on the beam but can sit on it; 1 point, completely unable to crawl and cannot place hind limbs in horizontal position, falls off when placed on the beam.

### 3.4 Forelimb Placement Test (FPT) Score: The assessment ranges from 0 to 10 points, with higher scores indicating better neurological behavioral status. The scoring rules are as follows:

The vibrissae-evoked forelimb placement test (also known as the paw whisker test) can be used to assess asymmetry in the sensorimotor cortex and striatum. The examiner holds the dorsal skin of the rat with all four limbs suspended and brushes the vibrissae against the edge of a table corner to test the activity of the ipsilateral forelimb. Unimpaired animals can quickly place the forelimb on the tabletop, while this action is impaired to varying degrees in brain-injured animals. Each side of the rat is tested 10 times, and the percentage of times the forelimb touches the table edge is the score for that side. The rat should be held gently with forelimbs hanging freely; the rat should be gently moved up and down before the test to allow it to relax as much as possible. If the rat struggles, has muscle tension, or places its limbs on the examiner's hand, it is not counted.

### 4. TTC Staining and Infarct Volume Quantification

2,3,5-Triphenyltetrazolium chloride (TTC) is a lipophilic, light-sensitive complex. TTC staining is a conventional method for detecting ischemic occlusion in mammalian tissues such as the brain or heart in preclinical research.

After euthanasia, the whole brain is cut into 5 consecutive 3-mm-thick coronal slices. The ipsilateral (Ips) and contralateral (Con) sides are divided by the midline. After TTC staining, the non-infarcted area appears red, and the infarcted area appears white. Since the white infarcted area may show edema, correction is performed using the ipsilateral non-infarcted area. After photographing with a stereo microscope, the infarct area or non-infarct area of each brain slice is quantified using Image-Pro Plus analysis software, and the volume is calculated based on the area.

Formula for ipsilateral non-infarct volume: ${V}_{\left(Ips\left(non\right)\right)} = \left[\left(A1+A2\right)/2\right] * D \left({mm}^{3}\right)$ where V_{(Ips(non))} is the ipsilateral non-infarct volume (mm³) of each brain slice, A1 and A2 are the areas (mm²) of the ipsilateral non-infarct region on the rostral and caudal surfaces of the brain slice, and D is the slice thickness (mm). Similarly, the brain infarct volume of the contralateral hemisphere V_{Con} can be calculated. The total brain infarct volume is the sum of the infarct volumes of the five brain slices.

Considering that edema causes irregular left and right hemisphere volumes, the infarct volume ratio is corrected using the contralateral hemisphere volume. Infarct volume (%) = (Contralateral (to infarct) hemisphere volume - Ipsilateral non-infarct volume) / Contralateral (to infarct) hemisphere volume × 100% i.e., ${V}_{infarct} \left(%\right) = \left[\left({V}_{Con}-{V}_{\left.\left(Ips\left(non\right)\right)\right)}\right./{V}_{Con}\right] * 100 %$ where V_{infarct} (%) is the percentage of infarct volume, V_{(Ips(non))} is the ipsilateral non-infarct volume, and V_{Con} is the contralateral volume.

### 5. Quantification of Brain Atrophy Volume

After euthanasia, the whole brain is cut into 5 consecutive 3-mm-thick coronal slices. The infarct side and contralateral side are divided by the midline. After photographing with a stereo microscope, the hemisphere area of each brain slice is quantified using Image-Pro Plus analysis software, and the hemisphere volume is calculated based on the hemisphere area.

The brain atrophy volume ratio is corrected using the contralateral hemisphere volume. Atrophy volume ratio (%) = (Contralateral (to infarct) hemisphere volume - Ipsilateral hemisphere volume) / Contralateral (to infarct) hemisphere volume × 100% i.e., ${V}_{atrophy} \left(%\right) = \left({V}_{Con}-{V}_{Ips}\right) / {V}_{Con} * 100 %$ where V_{atrophy} is the atrophy volume. Here, V_{Ips} is the ipsilateral hemisphere volume; V_{Con} is the contralateral (to infarct) hemisphere volume.

### 6.Paraffin Sectioning of Brain Tissue

Brain slices fixed in 4% paraformaldehyde for more than 72 hours were placed in embedding cassettes, washed with running water for 4 hours, and then placed in a tissue processor sequentially soaked in 75% ethanol for 2 hours, 95% ethanol for 2 hours, 100% ethanol for 2 hours, xylene for 2 hours, and paraffin for 2 hours. Tissues were then taken and embedded using a paraffin embedding machine. Glass slides were soaked in acetone solution containing 2% APES for 30 seconds, rinsed twice in distilled water after ,taking it out and dried in a fume hood. The paraffin-embedded tissues were cut into 4 µm sections using a microtome and smoothly attached to glass slides for subsequent histological staining and observation.

### 7. Hematoxylin and Eosin (HE) Staining

HE staining is a commonly used tissue staining method for observing pathological tissue structure. Hematoxylin stain is basic, and mainly used for staining chromatin in the nucleus and nucleic acids in the cytoplasm purplish-blue; eosin is an acidic dye, mainly used for staining cytoplasm and components in the extracellular matrix red.

Paraffin sections of brain tissue were placed on a slide warmer set at 65°C and heated for 60 minutes. The tissue sections were then sequentially subjected to xylene for 15 minutes, absolute ethanol for 5 minutes, 95% ethanol for 5 minutes, and 75% ethanol for 5 minutes, then rinsed with distilled water for 5 minutes. Subsequently, the tissue sections were placed in hematoxylin staining solution for 15 minutes, rinsed with running water for 5 minutes, differentiated in 1% hydrochloric acid-ethanol differentiation solution for 3-5 seconds (adequacy of differentiation degree was checked under a microscope), and rinsed with running water for 5 minutes. They were then placed in eosin staining solution for 5 minutes, and quickly rinsed with running water for 1 second after being taken out. The tissue sections were then sequentially subjected to 75% ethanol for 30 seconds, 95% ethanol for 5 minutes, absolute ethanol for 5 minutes, and xylene for 15 minutes. The tissue sections were taken out and mounted with neutral resin. Images of brain tissue sections were obtained using a pathological slide fluorescence scanner to capture staining images of the whole brain, cortex, and striatum.

### 8.Nissl Staining

Nissl staining is a staining method that evaluates the number of neurons by staining Nissl bodies in the neuronal cytoplasm. After staining, neurons appear purplish-blue and are commonly used to display the basic neural structure of the brain or spinal cord. Larger and more numerous Nissl bodies in neurons indicate stronger protein synthesis ability of the nerve cells; conversely, when nerve cells are damaged, the number of Nissl bodies may decrease or even disappear.

Paraffin sections of brain tissue were placed on a slide warmer set at 65°C and heated for 60 minutes. The tissue sections were then sequentially subjected to xylene for 15 minutes, absolute ethanol for 5 minutes, 95% ethanol for 5 minutes, and 75% ethanol for 5 minutes, then rinsed with distilled water for 5 minutes. Subsequently, the tissue sections were stained with Nissl staining solution for 3-10 minutes, quickly washed twice with distilled water, then soaked in absolute ethanol for 5 s, cleared in xylene for a few seconds, taken out, and mounted with neutral resin. Images of brain tissue sections were obtained using a pathological slide fluorescence scanner to capture staining images of the whole brain, cortex, and striatum. Finally, ImageJ software was used to count the number of cells in each region.

### 9.Bliss Independence Statistical Model

The definition of drug synergy is crucial in the fields of pharmacology, toxicology, and medicine. Drug synergy refers to the enhanced effect achieved when two or more drugs are used in combination.

To investigate the synergistic effect of MLB and Rg1 in treating ischemic stroke, the Bliss independence statistical model was used. Using infarct volume as an example to illustrate the statistical method. Firstly, the following calculations were performed: V_{MCAO}, V_{MLB}, V_{Rg1}, and V_{MLB/Rg1} represent the infarct volumes of the MCAO, MLB, Rg1, and MLB/Rg1 groups, respectively. I_{MLB}, I_{Rg1}, and I_{MLB/Rg1} represent the ratios of infarct volume in the MLB, Rg1, and MLB/Rg1 groups to the MCAO group. Iᵥₐₗᵤₑₛ were calculated using Formulas 1, 2, and 3. ${I}_{MLB} = \left({V}_{MLB}/{V}_{MCAO}\right) \times 100 %$ (Formula 1) ${I}_{Rg 1} = \left({V}_{Rg 1}/{V}_{MCAO}\right) \times 100 %$ (Formula 2) ${I}_{MLB / Rg 1} = \left({V}_{MLB / Rg 1}/{V}_{MCAO}\right) \times 100 %$ (Formula 3)

The Bliss independence statistical model was used to evaluate the synergistic effect of MLB and Rg1 in treating ischemic stroke. Independence is the theoretical value of synergy calculated for MLB and Rg1. According to the Bliss independence statistical model, Independence is calculated as shown in Formula 4. $Independence = {I}_{MLB} \times {I}_{Rg 1}$ (Formula 4)

To simplify the statistical test of synergy, after testing whether the data conforms to a normal distribution using the Kolmogorov-Smirnov test, a log-scale transformation was performed to obtain the additive equation as shown in Formula 5. ${InV}_{SaLB} + {InV}_{Rg 1} - {InV}_{MCAO} - {InV}_{Sa 1 B} {/}_{Rg 1} > 0$ (Formula 5)

Based on statistical methods, one-way ANOVA with Tukey's multiple comparison test was used to analyze differences between groups. If Formula 5 holds true and there is a statistically significant difference between I_{MLB/Rg1} and Independence, MLB and Rg1 are considered synergistic.

### 10. Small Animal Magnetic Resonance Imaging (MRI)

MRI data were acquired on an 11.7 T BioSpec 117/16 USR MRI system. A 2 × 2 phased array coil and a 72 mm volume resonator were used for transmission and image acquisition, respectively. All animals were anesthetized with 1% to 1.5% isoflurane, and their body temperature was maintained at 37°C to ensure proper fixation during MRI scanning. Respiration rate was monitored and maintained at 95 - 110 breaths per minute.

T2-weighted imaging (T2WI): Whole-brain T2WI was obtained using a rapid acquisition with relaxation enhancement (RARE) sequence with high resolution, flip angle 90°, repetition time (TR) 2500 ms, echo time (TE) 6.5 ms, and spatial resolution 0.063 × 0.063 × 0.5 mm³. For T2AMP, a multi-slice multi-echo (MSME) sequence was used with a flip angle of 90°, TR 2200 ms, and 30 effective TEs ranging from 7.5 ms to 225 ms.

Susceptibility Weighted Imaging (SWI): For SWI, a flow-compensated fast low-angle shot (FLASH) sequence was used to detect vascular abnormalities, with TR = 350 ms, TE = 12 ms, matrix size = 200 × 200, spatial resolution = 0.08 × 0.08 mm², slice thickness = 0.8 mm.

Arterial spin labeling (ASL): ASL images were acquired using a drift-corrected steady pulsed imaging and labeling (dSPIL) sequence with TE = 3.0 ms, TR = 5000 ms, and 32 time repetitions. Image Analysis and Data Processing: Motion correction and adjustment were performed using SPMMouse based on SPM8 (http://www.fil.ion.ucl.ac.uk/spm/software/spm8/), and data analysis was performed using MATLAB 2021b (MathWorks, Natick, USA). Other MRI modalities were rigidly co-registered to T2WI. A least squares algorithm was used to fit the signal intensity data to a mono-exponential decay curve, and T2-relaxation times for the whole brain were calculated based on T2MAP. Cerebral blood flow (CBF) images were processed using custom programs written in MATLAB. Regions of interest (ROIs) under different MRI modalities were manually created using ITK-SNAP software. Cerebral blood flow evaluation method: The brain tissue was divided into infarct core, infarct border zone, and contralateral areas, and the CBF values within the regions were measured.

### 11. Detection of Activated Partial Thromboplastin Time (APTT)

Activated partial thromboplastin time (APTT) was measured using a four-channel semi-automated coagulation analyzer (MC-4000, German M.C. Co., Ltd.) following the instrument's instructions. The APTT assay kit was purchased from Beijing Wokai Biotechnology Co., Ltd.; reaction cuvettes were purchased from Taizhou Kangjin Medical Devices Co., Ltd., KB005. Operations were performed according to the instrument and reagent instructions.

### 12. Detection of Cardiac Function

The MPA cardiac function analysis system (ALC-MPA, Shanghai ALCOT Biotech Co., Ltd.) can monitor, record, store, and analyze changes in cardiac function and hemodynamics in real-time via sensors. Before the experiment, the PE catheter and pressure transducer were filled with physiological saline without any air bubbles. If bubbles were present, they were expelled by gently flicking the catheter while pushing the syringe with water to flush them out. After anesthesia, the neck to mandible area of the animal was shaved with clippers, the animal was tied to a board with a rope, the skin on the right side of the neck was disinfected with an iodophor cotton ball and incised. The subcutaneous connective tissue between the bilateral bulging glands was separated along the midline, and the anterior cervical muscles between the right muscle masses were separated. Vessels and nerves around the common carotid artery (CCA) were carefully peeled off, and isolated the CCA. The distal end of the CCA was ligated with 4-0 suture, the proximal end was clamped with a vascular clip, and a thread was left around the proximal end. The proximal three-way stopcock was opened, the distal three-way stopcock was closed, and the PE catheter filled with 10% heparin sodium solution was placed flat on the table. A small opening was cut in the CCA, the PE catheter was inserted, the proximal suture was tied around the artery with the PE catheter inserted, and the vascular clip was opened to continue inserting the PE catheter to collect cardiac function indicators.

### 13. Detection of Blood Oxygen Saturation

A PowerLab system (PowerLab 8/30, AD Instruments) was used. An oximeter and an oximetry probe were connected. The infrared probe of the oximeter was clipped to the left hind paw pad of the animal, and data acquisition was performed according to the instrument instructions.

### 14. Animal Exclusion Criteria

Animals that died due to surgical reasons before drug administration;
Animals that died due to subarachnoid hemorrhage after drug administration; Animals with failed modeling: animals with infarct volume less than 10% due to insufficient filament insertion length or animals where the filament did not enter the internal carotid artery during modeling.

### 15. Animal Addition Criteria

Animals meeting the exclusion criteria were replaced with an equivalent number of animals to ensure a final group size of 8 animals.

### 16. Data Statistics

Data are presented as x ± S.E.M. After confirming homogeneity of variance, one-way ANOVA with Tukey's multiple comparison test was used to compare differences among multiple groups. A t-test was used to compare differences between two groups. Rank-sum test was used to compare ordered data such as Longa scores and Garcia scores. Synergy was analyzed using the Bliss non-independent model. P < 0.05 was considered statistically significant.

### Preparation Examples

### Preparation Methods for Magnesium Salvianolate B, Calcium Salvianolate B, and Free Salvianolic Acid B

**1. Extraction of Free Salvianolic Acid B (SalB):** 200 g of Salvia miltiorrhiza tablets were ultrasonicated twice with 10 volumes of deionized water. The aqueous solutions were combined and filtered through a Buchner funnel (diameter 30 cm, filtrate flask 10 L) packed with normal phase silica gel. After filtration, the silica gel was washed with a small amount of deionized water, and the aqueous solutions were combined, totaling approximately 5 L. The aqueous solution was loaded onto a medium-pressure C18 reverse-phase chromatographic column (60*450 mm) and eluted with a gradient of 10%, 20%, and 30% methanol, each with 1000 mL, collecting one fraction for every 100mL.
   Each fractions were detected by HPLC. HPLC conditions: acetonitrile-0.3% phosphoric acid water (30:70), detection wavelength: 286 nm. The retention time of salvianolic acid B was at approximately 8 minutes. Fractions containing salvianolic acid B were combined. The eluate was concentrated and loaded onto a high-pressure preparative HPLC column for separation and preparation of salvianolic acid B using acetonitrile:0.3% phosphoric acid water (30:70) as the mobile phase, detection wavelength 286 nm. Eluates containing salvianolic acid B with acceptable purity were collected and concentrated in a rotary evaporator at 40°C to remove acetonitrile. At this point, approximately 2 L of free salvianolic acid B solution was prepared. This solution was loaded onto a C18 reverse-phase column (15*100 mm) and eluted with pure methanol, yielding approximately 50 mL of concentrated solution, which was concentrated on a rotary evaporator at 40°C to remove methanol and dissolved to to 50 mL with a small amount of water. After lyophilization, pure free salvianolic acid B was obtained (light yellow powder).
**2. Preparation of Magnesium Salvianolate B (MLB):** 60 mg of magnesium carbonate was dissolved in 1000 mL of deionized water by heating and stirring to accelerate dissolution, with a heating temperature of about 80°C. After cooling, it was stored in a refrigerator at about 4°C. The above solution containing free salvianolic acid B was added to the magnesium carbonate solution with stirring.
   The magnesium salvianolate B solution at the end of the reaction was loaded onto a medium-pressure C18 reverse-phase chromatographic column (60*450 mm) and eluted with a gradient of pure water 1000 mL, 10% methanol solution 1000 mL, 15% methanol solution 2000 mL, and 20% methanol solution 1000 mL, collecting one fraction per 100 mL. Each fraction was detected by HPLC. Fractions with acceptable purity of magnesium salvianolate B were combined and concentrated on a rotary evaporator at 40°C to remove methanol. The methanol-removed magnesium salvianolate B solution was again loaded onto a medium-pressure C18 reverse-phase column (40*400 mm) and eluted with a gradient of pure water 500 mL, 10% methanol solution 500 mL, and 20% methanol solution 500 mL. Each Fraction was detected by HPLC. Fractions with acceptable purity of magnesium salvianolate B were combined, totaling approximately 500 mL, and concentrated on a rotary evaporator at 40°C. This concentrate was frozen in a -30°C freezer, and lyophilized for 2-3 days the next day. After lyophilization, it was further dried in a vacuum drying oven at 40°C for 48 hours. HPLC purity was 99.1%.
**3.Preparation of Calcium Salvianolate B (SalB-Ca):** 100 mg of calcium bicarbonate was dissolved in 1000 mL of deionized water by heating and stirring to accelerate dissolution, with a heating temperature of about 80°C. After cooling, it was stored in a refrigerator at about 4°C. The above solution containing free salvianolic acid B was added to the calcium bicarbonate solution with stirring.
   The calcium salvianolate B solution at the end of the reaction was loaded onto a medium-pressure C18 reverse-phase chromatographic column (60*450 mm) and eluted with a gradient of pure water 1000 mL, 10% methanol solution 1000 mL, 15% methanol solution 2000 mL, and 20% methanol solution 1000 mL, collecting one fraction every 100 mL. Each fraction was detected by HPLC. Fractions with acceptable purity of calcium salvianolate B were combined and concentrated on a rotary evaporator at 40°C to remove methanol. The methanol-removed calcium salvianolate B solution was again loaded onto a medium-pressure C18 reverse-phase column (40*400 mm) and eluted with a gradient of pure water 500 mL, 10% methanol solution 500 mL, and 20% methanol solution 500 mL. Each fraction was detected by HPLC. Fractions with acceptable purity of calcium salvianolate B were combined, totaling approximately 500 mL, and concentrated on a rotary evaporator at 40°C to a minimum. This concentrate was frozen in a -30°C freezer, and lyophilized for 2-3 days the next day. After lyophilization, it was further dried in a vacuum drying oven at 40°C for 48 hours. HPLC purity was 99.5%.
(4)-OES refers to inductively coupled plasma emission spectrometry, which can be used for qualitative and quantitative analysis of more than seventy metal elements and some non-metal elements in samples from geology, environmental protection, chemical industry, biology, medicine, food, metallurgy, agriculture, and other fields.

ICP-OES detection results are as follows:

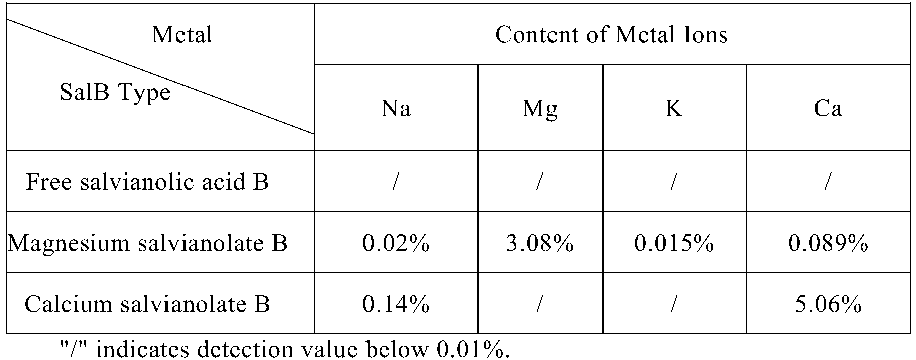

### Example 1: Comparative Study of the Effects of Free Salvianolic Acid B (SalB), Magnesium Salvianolate B (MLB), and Calcium Salvianolate B (SalB-Ca)

The permanent cerebral ischemia animal model was used to investigate the acute phase protection of different combinations in rats within 24 hours of ischemia. Simulating the clinical application scenario, test substances were administered via tail vein injection 3 hours after middle cerebral artery occlusion according to the experimental animal grouping and dosing table, with a test substance volume of 10 ml/kg. Tissue samples were collected at 24 hours after surgery. This experiment was conducted in a double-blind manner. The injection reagents required for the experiment were prepared before the start of the experiment, and blinding was not broken until all indicators were statistically analyzed. The weight ratio of SalB, MLB, and SalB-Ca to Rg1 was all 5:2.

| Group (n=8) | Injection Reagent |
|---|---|
| Sham group | 0.9% Sodium Chloride Injection |
| MCAO group | 0.9% Sodium Chloride Injection |
| SalB/Rg1 group | Free SalB/Rg1 combination (10 mg/kg) |
| MLB/Rg1 group | Magnesium SalB/Rg1 combination (10 mg/kg) |
| SalB-Ca/Rg1 group | Calcium SalB/Rg1 combination (10 mg/kg) |

### 1. Survival Rate

The survival rates of the Sham, MCAO, free SalB/Rg1, SalB-Ca/Rg1, and MLB/Rg1 groups were 100%, 71.4%, 87.5%, 75%, and 100%, respectively.

### 2. Infarct Volume

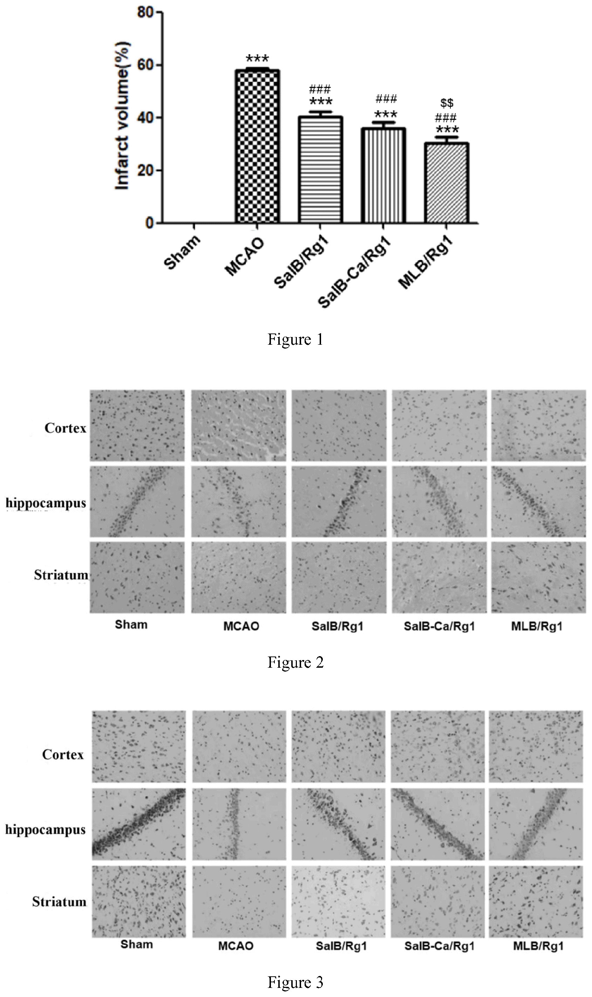

Compared with the MCAO model group (57.7 ± 2.8%), the infarct volumes of the free SalB/Rg1 group (40.1 ± 6.4%, P<0.001), SalB-Ca/Rg1 group (35.8 ± 7.0%, P<0.001), and MLB/Rg1 group (30.4 ± 5.9%, P<0.001) were significantly reduced. Surprisingly, compared with the free SalB/Rg1 group, the infarct volume of the SalB-Ca/Rg1 group was further reduced, but the difference was not significant; while the MLB/Rg1 group showed a significantly further reduction in infarct volume compared with the free SalB/Rg1 group (40.1 ± 6.4% versus 30.4 ± 5.9%, P<0.01), indicating that the ability of calcium and magnesium salts of salvianolic acid B to penetrate the blood-brain barrier and exert therapeutic effects is superior to free salvianolic acid B. Among them, MLB/Rg1 had a more excellent effect in reducing infarct volume and treating stroke (Figure 1).

The above results indicate that compared with free SalB/Rg1, the use of salts of salvianolic acid provides better brain protection, especially the survival rate of the MLB/Rg1 group can reach 100%, which is of great significance for reducing mortality from cerebral infarction. Those skilled in the art understand that for the treatment of brain diseases, drugs need to penetrate the blood-brain barrier to exert their effects. The drugs of the present invention also need to penetrate the blood-brain barrier after intravenous injection to reach the infarct area. Surprisingly, the SalB salts of the present invention have lower lipophilicity compared with free SalB (usually making it more difficult to penetrate the blood-brain barrier), but experiments have proven that the SalB salt/Rgl combination has significantly higher brain protection and therapeutic effects, which is extremely unexpected to those skilled in the art.

### 3. HE Staining

Middle cerebral artery occlusion leads to necrosis or apoptosis of neurons in the corresponding areas of the rat brain cortex and striatum. We analyzed the morphology and structural changes of neurons in the cerebral cortex and striatum using HE staining. Figure 2 shows representative images of HE staining of coronal brain sections. In the Sham group, the nerve cells in the cortex and striatum were densely arranged, with intact structure and normal morphology. In the MCAO group, due to cerebral ischemia, disorganized arrangement of nerve cells, neuronal necrosis, and pyknosis were observed in the cortex and striatum. Free SalB/Rg1 treatment showed a trend of improving the pathological tissue abnormalities after ischemic stroke. Compared with free SalB/Rg1 or SalB-Ca/Rg1, the morphology and structure of nerve cells in the cortex and striatum of the MLB/Rg1 group were closer to those of the Sham group, indicating that MLB/Rg1 has a better protective effect on nerve cells after cerebral ischemia.

### 4. Nissl Staining

We detected the number of surviving neurons in the cerebral cortex, striatum, and hippocampus using Nissl staining. Figure 3 shows representative images of Nissl staining of coronal brain sections. It can be seen that compared with the Sham group, the number of surviving neurons in the cerebral cortex, striatum, and hippocampus was significantly reduced in the MCAO group. Compared with the MCAO group, the number of neurons in the cortex, striatum, and hippocampus were significantly increased in the MLB/Rg1 group; and compared with the number of neurons in the cortex, striatum, and hippocampus in the free SalB/Rg1 and SalB-Ca/Rg1 groups, the MLB/Rg1 group showed a stronger trend in protecting neurons, suggesting that MLB/Rg1 has the potential to improve the behavioral function of animals after stroke.

### Example 2: Synergistic Effect Study of Magnesium Salvianolate B (MLB) Combined with Ginsenoside Rg1 in Treating Ischemic Stroke

To clarify whether SalB magnesium salt (MLB) and Rg1 have a synergistic effect, the experiment was strictly designed according to the requirements of the Bliss independence statistical model for synergy evaluation. Briefly, using the permanent cerebral ischemia animal model, MLB and Rg1 were combined at a ratio of 5:2, and the subacute phase (7 days of ischemia) was evaluated. Rats were randomly divided into a sham-operated group (Sham), a model control group (MCAO), an MLB group, an Rg1 group, and an MLB/Rg1 combination group according to a random allocation table, with n = 10 per group. The Sham and MCAO groups received 0.9% Sodium Chloride Injection, the MLB group received 7.14 mg/kg SalB magnesium salt, the Rg1 group received 2.86 mg/kg Rg1, and the MLB/Rg1 group received 10 mg/kg MLB/Rg1.

Simulating the clinical application scenario, test substances were administered via tail vein injection 3 hours after middle cerebral artery occlusion according to the experimental animal grouping and dosing table, with an injection volume of 10 ml/kg, once daily, for 7 consecutive days before tissue sampling. This experiment was conducted in a double-blind manner. The injection reagents required for the experiment were prepared before the start of the experiment, and blinding was not broken until all indicators were statistically analyzed.

| Group (n=10) | Injection Reagent |
|---|---|
| Sham group | 0.9% Sodium Chloride Injection |
| MCAO group | 0.9% Sodium Chloride Injection |
| MLB/Rg1 group | Magnesium salvianolate B/Rgl (10 mg/kg) |
| MLB group | Magnesium salvianolate B (7.14 mg/kg) |
| Rg1 group | Rg1 (2.86 mg/kg) |

### 1. MLB and Rg1 Have a Synergistic Effect in Reducing Infarct Volume

Although the efficacy of the SalB magnesium salt/Rgl combination (MLB/Rg1) was previously evaluated in the MCAO rat model, whether MLB/Rg1, as a combination, has a synergistic effect in treating ischemic stroke has not been clarified, and it is a question that must be answered before clinical translation of MLB/Rg1. The Bliss independence statistical model is an important method for evaluating the interaction of multi-component drugs. By establishing a rat permanent middle cerebral artery ischemia model, the Bliss independence statistical model was used to clarify the synergistic effect of MLB and Rg1 by evaluating cerebral infarct volume.

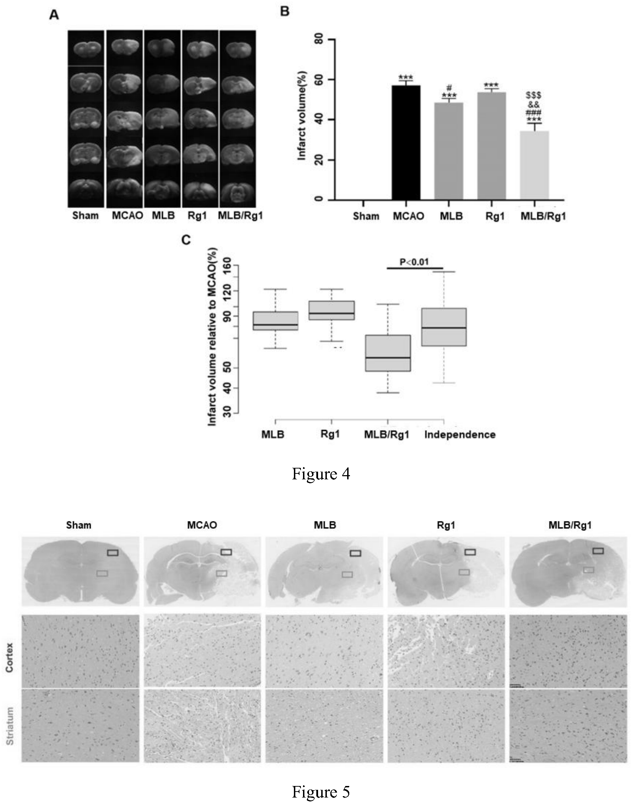

As shown in Figure 4(A), after TTC staining, the non-infarcted area of the brain appeared red, and the infarcted area appeared white. Quantitative results of cerebral infarct volume are shown in Figure 4(B). Compared with the Sham group, the MCAO group showed a significant increase in infarct volume (57.1 ± 6.6% versus 0 ± 0%, P <0.001), indicating the successful establishment of the permanent cerebral infarction model in rats. Compared with the MCAO group (57.1 ± 6.6%), the MLB group (48.4 ± 5.6%) and the Rg1 group (53.0 ± 3.8%) showed a decreasing trend, but without statistical significance; the MLB/Rg1 group (34.4 ± 9.3%, P <0.001) significantly reduced infarct volume, and MLB/Rg1 still significantly enhanced the reduction of infarct volume compared with the MLB group (P<0.01) and the Rg1 group (P<0.001).

As shown in Figure 4(C), according to the Bliss independent statistical model, the percentage of infarct volume of each treatment group relative to the MCAO group was plotted. The data of each group conformed to a normal distribution after the Kolmogorov-Smirnov test and met homogeneity of variance after the F-test. Relative to the MCAO group, the infarct volume percentage of the MLB/Rg1 group was 61.2 ± 16.9%, the infarct volume percentage of the MLB group was 85.9 ± 13.4%, and the infarct volume percentage of the Rg1 group was 95.2 ± 13.4%. According to the Bliss independent statistical model formula, the theoretical effect of the combination of the two calculated based on the MLB and Rg1 groups, i.e., the infarct volume percentage of Independence group, was 82.7 ± 21.7%. Compared with the Independence group, the MLB/Rg1 group significantly reduced infarct volume (P < 0.01). Therefore, Bliss independent statistical analysis indicated that MLB and Rg1 have a significant synergistic effect in reducing infarct volume.

### 2. Compared with MLB or Rg1 Alone, MLB/Rg1 Enhances the Neuroprotective Effect

Middle cerebral artery occlusion leads to necrosis or apoptosis of nerve cells in the corresponding areas of the rat brain cortex and striatum. We analyzed the morphology and structural changes of nerve cells in the cerebral cortex and striatum using HE staining. Figure 5 shows representative images of HE staining of coronal brain sections, where the red square area indicates the cortex and the blue square area indicates the striatum. In the Sham group, the nerve cells in the cortex and striatum were densely arranged, with intact structure and normal morphology. In the MCAO group, due to cerebral ischemia, disorganized arrangement of nerve cells, neuronal necrosis, and pyknosis were observed in the cortex and striatum. MLB or Rg1 treatment showed a trend of improving the pathological tissue abnormalities after ischemic stroke. Compared with MLB or Rg1 alone, the morphology and structure of nerve cells in the cortex and striatum of the MLB/Rg1 group were closer to those of the Sham group, indicating that MLB/Rg1 has a better protective effect on nerve cells after cerebral ischemia.

### 3. Compared with MLB or Rg1 Alone, MLB/Rg1 Improves Neurological Function

Ischemic stroke is a highly disabling disease. Necrosis or apoptosis of neurons leads to the occurrence of vascular dementia. Vascular dementia mainly manifests as cognitive dysfunction, including decrease in attention, motor ability, and ability to analyze situations and make decisions. The cerebral cortex is the highest center for controlling somatic movement, and the striatum primarily regulates muscle tone and coordinates various fine and complex movements. Evaluating the severity of neurological dysfunction in patients through behavioral assessment is one of the important methods for clinical diagnosis. Therefore, on day 1, 3, 5, and 7 after MCAO surgery, we evaluated the neurological function and neurological behavior of rats using Garcia JH scores and Longa scores.

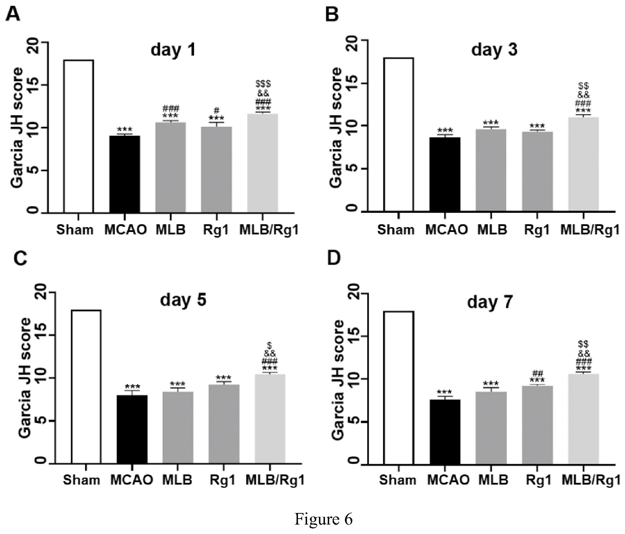

As shown in Figure 6, compared with the Sham group, the Garcia JH scores of the MCAO group were significantly reduced on day 1 (9.1 ± 0.6 versus 18 ± 0, P <0.001), day 3 (8.7 ± 0.8 versus 18 ± 0, P <0.001), day 5 (8.0 ± 1.2 versus 18 ± 0, P <0.001), and day7 (7.6 ± 0.9 versus 18 ± 0, P <0.001) after surgery, indicating severe impairment of motor and sensory functions in rats. MLB/Rg1 treatment significantly improved neurological dysfunction. Compared with MLB alone, MLB/Rg1 significantly upregulated Garcia JH scores on day 1 (11.6 ± 0.7 versus 10.6 ± 0.7, P <0.01), day 3 (11.0 ± 0.8 versus 9.6 ± 0.7, P <0.01), day 5 (10.4 ± 0.5 versus 8.4 ± 1.3, P <0.01), and day 7 (10.6 ± 0.5 versus 8.5 ± 1.0, P <0.01); compared with Rg1 alone, MLB/Rg1 significantly upregulated Garcia JH scores on day 1 (11.6 ± 0.7 versus 10.1 ± 1.4, P <0.001), day 3 (11.0 ± 0.8 versus 9.3 ± 0.5, P <0.01), day 5 (10.4 ± 0.5 versus 9.2 ± 0.8, P <0.05), and day 7 (10.6 ± 0.5 versus 9.2 ± 0.4, P <0.001), indicating that MLB/Rg1 can improve neurological function in rats.

### 4. Compared with MLB or Rg1 Alone, MLB/Rg1 Improves Behavioral Capacity

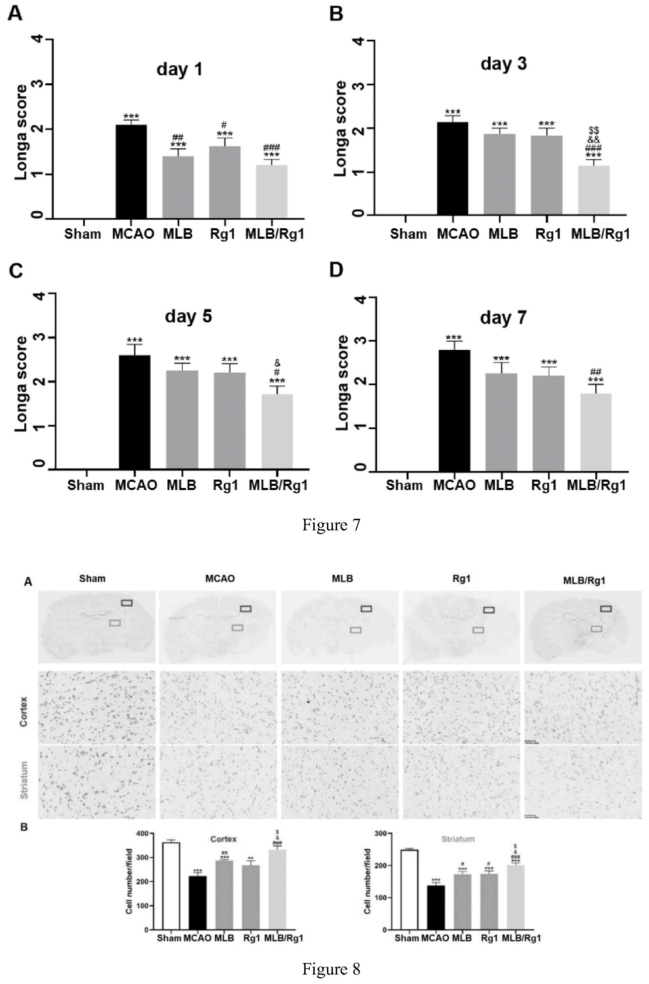

We comprehensively evaluated the behavioral capacity of rats using the Longa score. The results are shown in Figure 7, compared with the Sham group, the Longa scores of the MCAO group were significantly increased on day 1 (2.1 ± 0.3 versus 0 ± 0, P<0.001), day 3 (2.1 ± 0.4 versus 0 ± 0, P<0.001), day 5 (2.6 ± 0.5 versus 0 ± 0, P <0.001), and day 7 (2.8 ± 0.4 versus 0 ± 0, P<0.001) after surgery, indicating severe behavioral capacity deficits in rats. MLB/Rg1 treatment significantly improved behavioral capacity. Compared with MLB alone, MLB/Rg1 significantly reduced Longa scores on day 3 (1.1 ± 0.4 versus 1.9 ± 0.4, P <0.01) and day 5 (1.7 ± 0.5 versus 2.3 ± 0.5, P <0.05); compared with Rg1 alone, MLB/Rg1 significantly reduced Longa scores on day 3 (1.1 ± 0.4 versus 1.8 ± 0.4, P <0.01) and day 5 (1.7 ± 0.5 versus 2.2 ± 0.4, P <0.05). The results of the Longa score indicated that MLB/Rg1 can improve behavioral capacity in rats.

### 5. Compared with MLB or Rg1 Alone, MLB/Rg1 Enhances the Inhibiting effect on Neuronal Damage

We detected the number of surviving neurons in the cerebral cortex and striatum using Nissl staining. Figure 8 shows representative images of Nissl staining of coronal brain sections (*Panel* A) and quantitative results of surviving neuron numbers *(Panel B).* The quantitative results in Figure 8(B) showed that compared with the Sham group, the number of neurons in the cortex (223.6 ± 30.2 versus 363.2 ± 22.2, P <0.001) and striatum (138.2 ± 20.7 versus 249.2 ± 9.0, P <0.001) of the MCAO group was significantly reduced. Compared with MLB alone, the number of neurons in the cortex (332.4 ± 33.9 versus 287.6 ± 8.9, P < 0.05) and striatum (201.6 ± 14.7 versus 172.8 ± 19.6, P < 0.05) was significantly increased in the MLB/Rg1 group; compared with Rg1 alone, the number of neurons in the cortex (332.4 ± 33.9 versus 267.8 ± 42.1, P < 0.05) and striatum (201.6 ± 14.7 versus 174.2 ± 20.9, P < 0.05) was significantly increased in the MLB/Rg1 group, showing a stronger trend in protecting neurons, suggesting an association with the improvement of behavioral function in rats after stroke by MLB/Rg1.

### Example 3: Study on the Synergistic Inhibition of Magnesium Salvianolate B (MLB) Combined with Ginsenoside Rg1 on Vascular Dementia

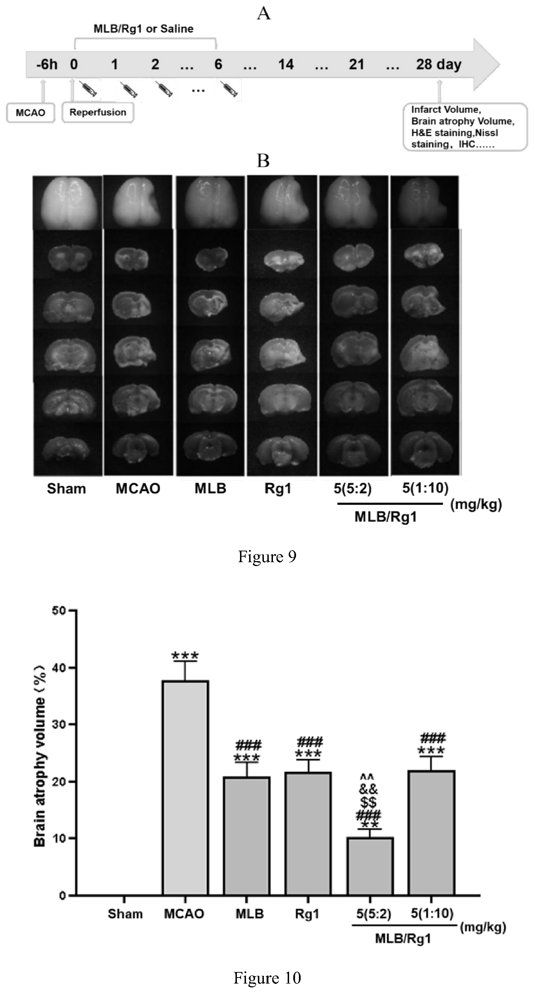

The reperfusion cerebral ischemia animal model was used to induce pathological changes of vascular dementia through long-term rearing after injury. SD rats with qualified neurological behavior were randomly divided into groups based on body weight, with 10 rats per group. The body weights of the animals after grouping were verified to ensure no differences between groups. Using the reperfusion cerebral ischemia model, reperfusion was perfomed at 6 h after middle cerebral artery infarction, and drugs were administered immediately thereafter, followed by daily administration once a day for 7 days via tail vein injection according to the grouping and dosing table. After 7 days of administration, animals were continuously fed until sampling on day 28. To meet the energy requirements of animals unable to eat due to modeling, 4 ml of enteral nutrition suspension was administered by gavage daily after modeling. Behavioral evaluations were performed on day 1, 3, 5, 7, 14, 21, and 28 after surgery. The experimental protocol is shown in Figure 9A.

| Group (n=10) | Injection Reagent |
|---|---|
| Sham (sham-operated control) | 0.9% Sodium Chloride Injection |
| MCAO (model control) | 0.9% Sodium Chloride Injection |
| (1:10) MLB/Rg1 group | 5 mg/kg MLB/Rg1 (MLB:Rgl = 1:10) |
| (5:2) MLB/Rg1 group | 5 mg/kg MLB/Rg1 (MLB:Rg1 = 5:2) |
| MLB group | 3.57 mg/kg MLB |
| Rg1 group | 1.43 mg/kg Rg1 |

As shown in Figure 9B, whether in the overall images or TTC-stained sections of brain, the model group animals showed significant brain atrophy. After receiving different types of treatment, the degree of atrophy decreased to some extent.

To investigate the effect of MLB/Rg1 on long-term vascular dementia in the ischemia-reperfusion model, brain atrophy volume was quantified. After euthanasia, the whole brain was cut into 5 consecutive 3-mm-thick coronal slices. The infarct side and contralateral side were divided by the midline. After photographing with a stereo microscope, the hemisphere area of each brain slice was quantified using Image-Pro Plus analysis software, and the hemisphere volume was calculated based on the hemisphere area. The results are shown in Figure 10, compared with the normal control group, the model group showed a significantly increased atrophy volume (37.81 ± 6.64%), indicating that cerebrovascular occlusion led to obvious brain atrophy. Compared with the model control group, both 5 mg/kg MLB/Rg1 (5:2, 10.31 ± 2.95%, P<0.01) and 5 mg/kg MLB/Rg1 (1:10, 21.93 ± 5.51%) significantly reduced atrophy volume. Use of SalB magnesium salt alone (20.83 ± 5.61%) and Rg1 alone (21.93 ± 5.51%) also reduced atrophy volume. However, 5 mg/kg MLB/Rg1 (5:2) further significantly reduced atrophy volume compared with the MLB group (P<0.01) and Rg1 group (P<0.01), indicating that MLB and Rg1 have a significant synergistic effect in inhibiting brain atrophy, thereby treating vascular dementia. However, from the results of SalB magnesium salt/Rg1 (1:10), it can be seen that the synergistic effect of SalB magnesium salt and Rg1 is only achieved at a specific preferred ratio.

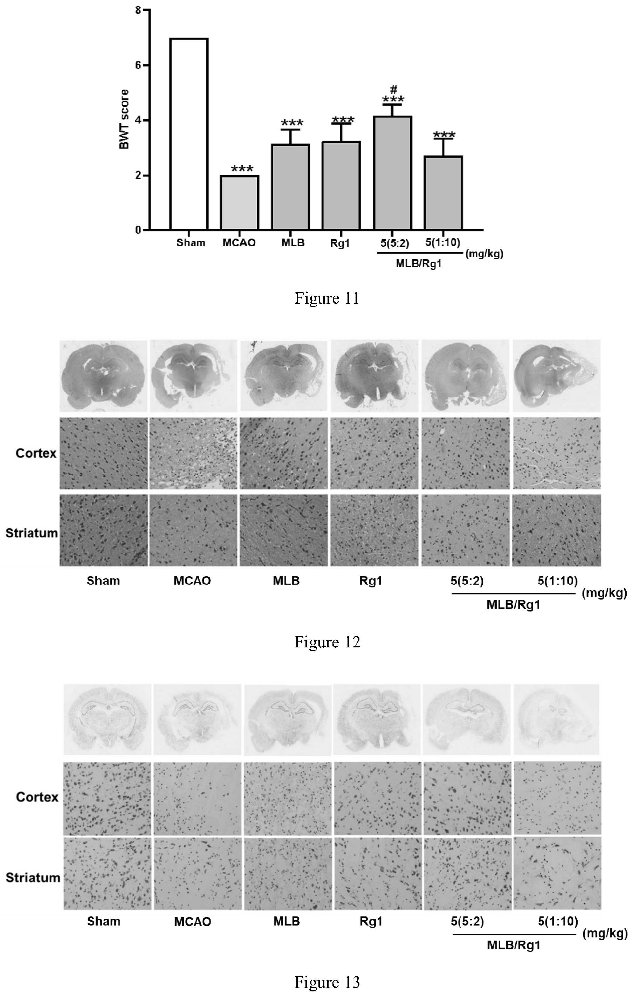

We conducted a comprehensive behavioral evaluation of the rats within 28 days after modeling. The results of the beam walking test on day 28 are shown in Figure 11. The MCAO model group showed the most severe behavioral function impairment; compared with the model group, all treatment groups improved behavioral function, but only the 5 mg/kg MLB/Rg1 (5:2) group showed a significant difference (Figure 11).

To evaluate the effect of the drugs on pathomorphology, HE staining was performed on sections (Figure 12). The results were consistent with the quantitative results of brain slice areas, with 5 mg/kg MLB/Rg1 (5:2) showing a reduction in atrophy volume and a decrease in necrotic cells at the injury site.

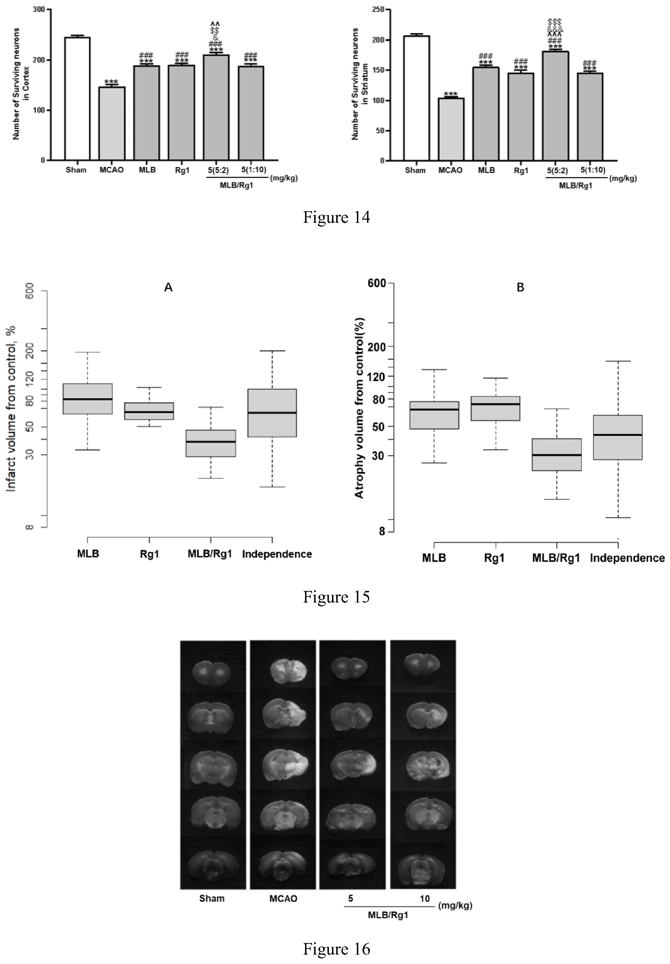

Nissl staining was used to evaluate neuronal morphology and number. Based on the Nissl staining results of the third brain slice (Figure 13), the model group showed obvious pyknosis and disappearance of Nissl bodies, and quantitative results (Figure 14) showed a significant reduction in neurons in both the cortex and striatum compared with the sham group. Compared with MLB, Rg1, and MLB/Rg1 (1:10), 5 mg/kg MLB/Rg1 (5:2) more significantly reduced neuronal death.

The Bliss method was used to evaluate the synergistic effect of MLB and Rg1 in reducing infarct volume and inhibiting brain atrophy. The results showed p<0.05, indicating a significant difference, suggesting that MLB/Rg1 (5:2) has a significant therapeutic effect in improving the progression of neurodegenerative diseases compared with MLB and Rg1, and that MLB and Rg1 have a synergistic effect (Figure 15).

Furthermore, further experimental results demonstrated that the SalB magnesium salt/Rgl (5:2) of the present invention was also significantly superior to free SalB/Rg1 (5:2) and SalB calcium salt/Rgl (5:2) in reducing vascular dementia induced by cerebral ischemia-reperfusion.

### Example 4: Study on the Inhibition of Magnesium Salvianolate B (MLB) Combined with Ginsenoside Rg1 Vascular Dementia

SD rats were randomly divided into groups based on body weight, and the body weights of the animals after grouping were tested for differences to ensure no differences between groups. Using the permanent middle cerebral artery ischemia animal model, drugs were administered 3 hours after infarction, followed by daily administration once a day for 7 days via tail vein injection according to the grouping and dosing table. Tissue samples were collected on day 28 after surgery. This experiment was conducted in a double-blind manner. Test substances were administered via tail vein injection at 3h, and on day 1, 2, 3, 4, 5, and 6 after surgery according to the grouping and dosing table, for a total of seven administrations. Behavioral evaluations were performed on day 1, 3, 5, 7, 14, 21, and 28 after surgery.

| Group (n=10) | Injection Reagent |
|---|---|
| Sham (sham-operated control) | 0.9% Sodium Chloride Injection |
| MCAO (model control) | 0.9% Sodium Chloride Injection |
| 5 mg/kg MLB/Rg1 group | 5 mg/kg MLB/Rg1 (SalB magnesium salt:Rg1 = |
| | 5:2) |
| 10 mg/kg MLB/Rg1 group | 10 mg/kg MLB/Rg1 (SalB magnesium salt:Rg1 = 5:2) |

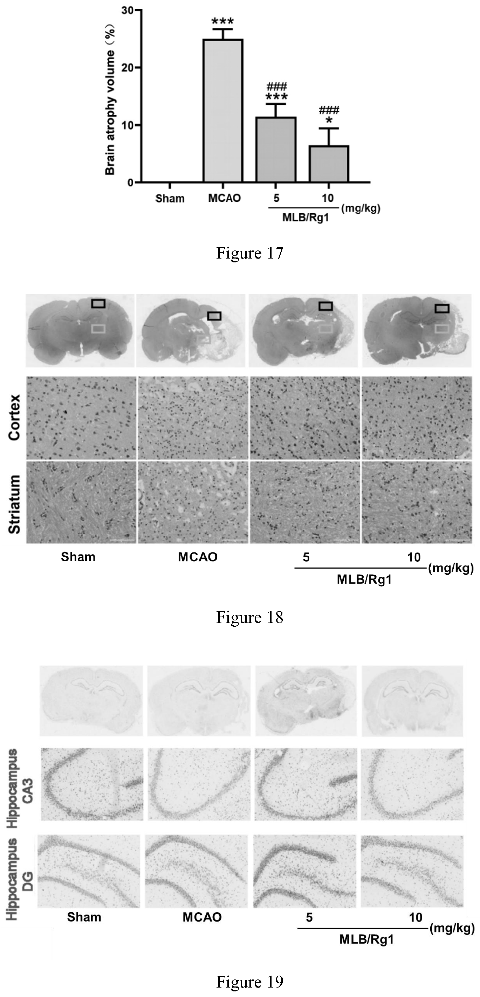

As shown in Figure 16, whether by overall images or TTC-stained sections of brain, the model group animals showed significant brain atrophy. After receiving different types of treatment, the degree of atrophy decreased significantly (Figure 17).

To evaluate the pathological improvement effect of MLB/Rg1 (5:2) on vascular dementia, HE staining was performed on brain sections. The results showed that, consistent with the quantitative results of brain slice areas, 5 mg/kg MLB/Rg1 showed a reduction in atrophy volume and a decrease in necrotic cells at the injury site (Figure 18).

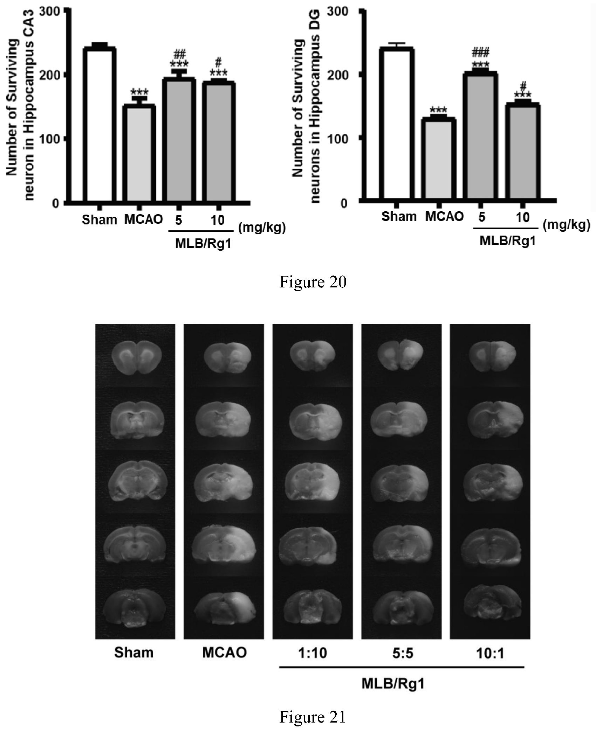

Since stroke may lead to long-term cognitive impairment, we chose the hippocampus, a brain region primarily responsible for learning and memory, to evaluate neuronal morphology and number using Nissl staining. Based on the Nissl staining results of the fourth brain slice (Figure 19), the model group showed obvious pyknosis and disappearance of Nissl bodies, and compared with the sham group, the model group showed a significant reduction in neurons. Compared with the model group, the MLB/Rg1 groups showed a significant reduction in neuronal death in the CA3 and dentate gyrus regions of the hippocampus (Figure 20).

Furthermore, further experimental results demonstrated that the MLB/Rg1 (5:2) of the present invention was also significantly superior to free SalB/Rg1 (5:2) and SalB-Ca/Rg1 (5:2) in reducing brain atrophy induced by permanent middle cerebral artery infarction.

### Example 5: Acute-Phase Protective Effect of Different Ratios of MLB and Rg1 on Stroke

SD rats with qualified behavioral assessments were randomly divided into groups based on body weight, with 10 rats per group. The body weights of the animals after grouping were verified to ensure no differences among groups. This experiment was conducted in a double-blind manner. Using the reperfusion cerebral ischemia model, reperfusion was performed at 6 h after infarction and drugs were administered immediately thereafter via tail vein injection, as a single dose. Tissue samples were collected at 24 h after reperfusion to quantify cerebral infarct volume.

| Group | Injection Reagent |
|---|---|
| Sham (sham-operated control) | 0.9% Sodium Chloride Injection |
| MCAO (model control) | 0.9% Sodium Chloride Injection |
| (1:10) MLB/Rg1 group | 5 mg/kg Magnesium salvianolate B/Ginsenoside Rg1 (1:10) |
| (5:5) MLB/Rg1 group | 5 mg/kg Magnesium salvianolate B/Ginsenoside Rg1 (5:5) |
| (10:1) MLB/Rg1 group | 5 mg/kg Magnesium salvianolate B/Ginsenoside Rg1 (10:1) |

Results: The down-regulation effects of different ratios of MLB/Rg1 on infarct volume were evaluated by TTC staining. The results showed that MLB/Rg1 (1:10), MLB/Rg1 (5:5), and MLB/Rg1 (10:1) reduced infarct volume to a similar extent, approximately 15% to 20% (Figure 21).

### Example 6: Acute-Phase Protective Effect of SalA Magnesium Salt and Rb1 Combination on Stroke

SD rats with normal neurological behavioral assessments were randomly divided into groups based on body weight, with 10 rats per group. The body weights of the animals after grouping were verified to ensure no differences among groups. This experiment was conducted in a double-blind manner. Using the reperfusion cerebral ischemia model, reperfusion was performed at 6h after infarction and drugs were administered via tail vein injection immediately thereafter, as a single dose. Behavioral evaluations were performed at 24 h after reperfusion, followed by tissue sampling to quantify cerebral infarct volume.

| Group | Injection Reagent |
|---|---|
| Sham (sham-operated control) | 0.9% Sodium Chloride Injection |
| MCAO (model control) | 0.9% Sodium Chloride Injection |
| SalA magnesium salt/Rb1 group | 5 mg/kg Magnesium salvianolate A/Ginsenoside Rb1 (5:5) |

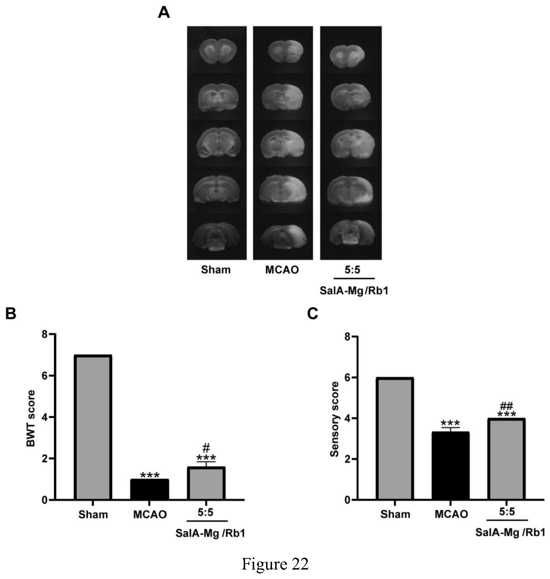

Results: The effect of SalA magnesium salt/Rb1 on reducing infarct volume was evaluated by TTC staining. The results showed that SalA magnesium salt/Rb1 reduced infarct volume by approximately 15%. From the behavioral evaluation, SalA-Mg/Rb1 significantly improved the Beam Walking Test (BWT) score (1.6 ± 0.2, P<0.05) and the sensory ability of Garcia JH (P<0.01) (Figure 22).

### Example 7: Acute-Phase Protective Effect of SalA Magnesium Salt and Rh2 Combination on Stroke

SD rats with normal behavioral assessments were randomly divided into groups based on body weight, with 10 rats per group. The body weights of the animals after grouping were verified to ensure no differences among groups. This experiment was conducted in a double-blind manner. Using the reperfusion cerebral ischemia model, reperfusion was preformed at 6h after infarction and drugs were administered via tail vein injection immediately thereafter, as a single dose. Behavioral evaluations were performed at 24 h after reperfusion, followed by tissue sampling to quantify cerebral infarct volume.

| Group | Injection Reagent |
|---|---|
| Sham (sham-operated control) | 0.9% Sodium Chloride Injection |
| MCAO (model control) | 0.9% Sodium Chloride Injection |
| SalA magnesium salt/Rh2 group | 5 mg/kg Magnesium salvianolate A/Ginsenoside Rh2 (10:1) |

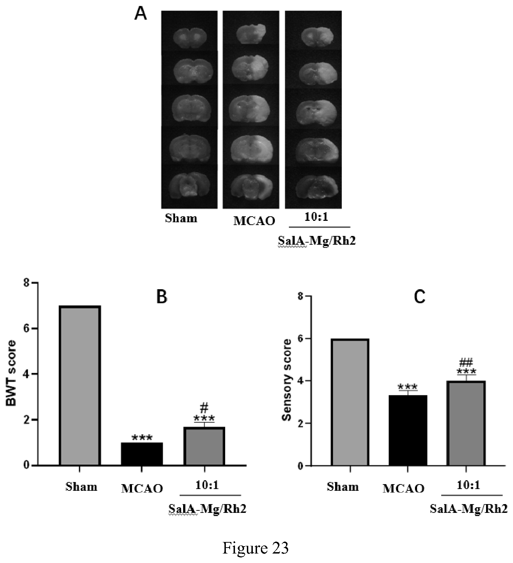

Results: The effect of the SalA magnesium salt/Rh2 group on reducing infarct volume was approximately 15% as evaluated by TTC staining. From the behavioral evaluation, SalA-Mg/Rh2 significantly improved the Beam Walking Test (BWT) score (1.7 ± 0.19, P<0.05) and the sensory ability of Garcia JH (4.0 ± 0.23, P<0.01) (Figure 23).

### Example 8: Acute-Phase Protective Effect of Different Ratios of Sodium Rosmarinate/Rb1 Combination on Stroke

SD rats with normal behavioral assessments were randomly divided into groups based on body weight, with 10 rats per group. The body weights of the animals after grouping were verified to ensure no differences among groups. This experiment was conducted in a double-blind manner. Using the reperfusion cerebral ischemia model, reperfusion was performed on 6h after infarction and drugs were administered via tail vein injection immediately thereafter, as a single dose. Behavioral evaluations were performed at 24 h after reperfusion, followed by tissue sampling to quantify cerebral infarct volume.

| Group | Injection Reagent |
|---|---|
| Sham (sham-operated control) | 0.9% Sodium Chloride Injection |
| MCAO (model control) | 0.9% Sodium Chloride Injection |
| RosA-Na/Rb1 group | 5 mg/kg Sodium rosmarinate/Ginsenoside Rb1 (1:10) |
| RosA-Na/Rb1 group | 5 mg/kg Sodium rosmarinate/Ginsenoside Rb1 (10:1) |

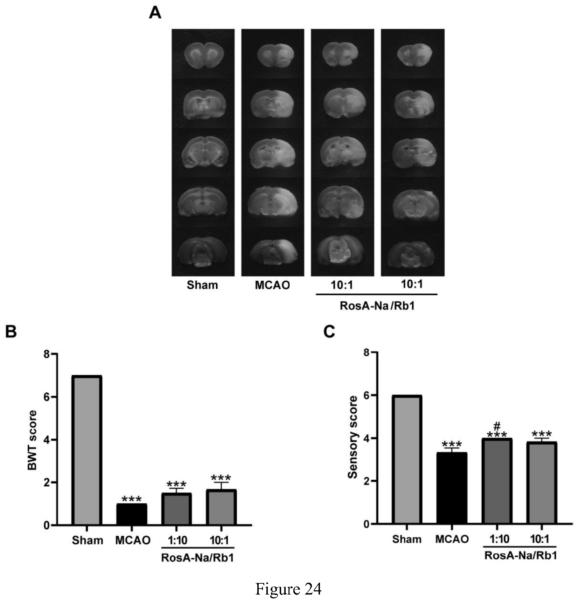

Results: The effect of different ratios of RosA-Na/Rb1 on reducing infarct volume was evaluated by TTC staining. The results showed that RosA-Na/Rb1 had a tendency to reduce infarct volume. Different ratios of RosA-Na/Rb1 showed a tendency to improve neurological behavior, and the (1:10) RosA-Na/Rb1 group significantly improved the sensory ability of Garcia JH (4.0 ± 0, P<0.05) (Figure 24).

### Example 9: Therapeutic Effect of Sublingual Administration of MLB/Rgl on Stroke

SD rats with normal behavioral assessments were randomly divided into groups based on body weight, with 10 rats per group. The body weights of the animals after grouping were verified to ensure no differences among groups. This experiment was conducted in a double-blind manner. Using the reperfusion cerebral ischemia model, reperfusion was performed at 6h after infarction and drugs were administered sublingually via drops thereafter, as a single dose. Behavioral evaluations were performed at 24 h after reperfusion, using the Longa score, Garcia JH score, forelimb placement test, and beam walking test. After behavioral evaluation, tissue samples were collected to quantify cerebral infarct volume.

| Group | Injection Reagent |
|---|---|
| Sham (sham-operated control) | 0.9% Sodium Chloride Injection |
| MCAO (model control) | 0.9% Sodium Chloride Injection |
| MLB/Rg1 sublingual administration group | 5 mg/kg Magnesium salvianolate B/Ginsenoside Rg1 (5:2) |

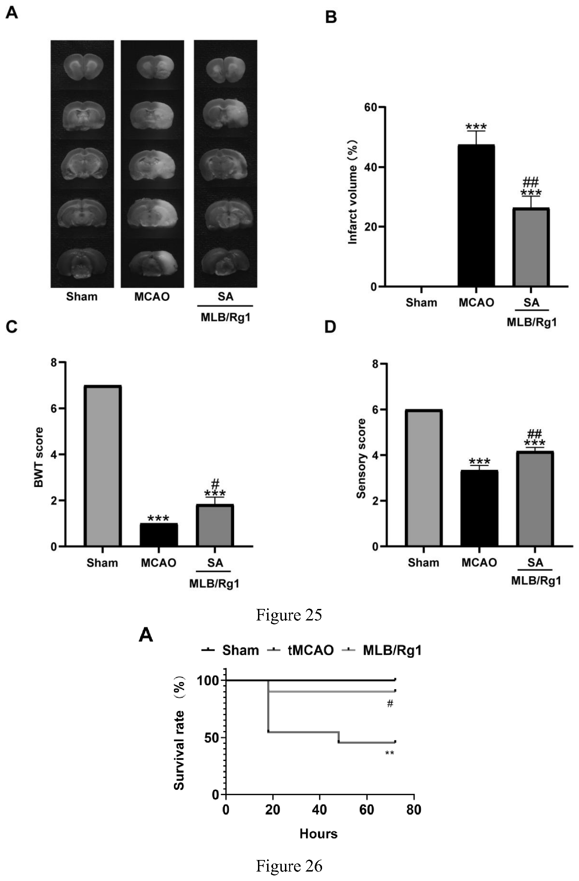

Results: infarct volume was evaluated by TTC staining, compared with the MCAO model group (47.4 ± 4.1), sublingual administration of MLB/Rg1 significantly reduced infarct volume (26.4 ± 3.6, p<0.01); significantly improved neurological behavior, and showed very significant improvement in the BWT score (P<0.05) and the sensory ability of Garcia JH (P<0.01) (Figure 25).

### Example 10: MLB/Rg1 Synergistically Inhibits Brain Edema and Improves Cerebral Blood Flow

The purity of magnesium salvianolate B and ginsenoside Rg1 was above 99% as determined by high-performance liquid chromatography. Magnesium salvianolate B and ginsenoside Rg1 were uniformly mixed at a ratio of 5:2 to obtain MLB/Rgl, which was then dissolved in physiological saline to the desired concentration and administered via tail vein injection according to the study protocol.

All mice were subjected to behavioral evaluation before the experiment to ensure normal neurological behavioral function. The reperfusion cerebral ischemia mouse model was used. The experiment included a sham-operated group, a model group, and an magnesium salvianolate B/ginsenoside Rg1 group (5 mg/kg MLB/Rgl). The MLB/Rg1 preparation was magnesium salvianolate B and Rg1 at a ratio of 5:2. To ensure objectivity, the experiment was conducted in a double-blind manner, and blinding was not broken until all experiments were completed and conclusions were drawn. All mice with normal neurological function were randomly divided into three groups (n = 10): sham-operated group (Sham), cerebral ischemia-reperfusion model group (tMCAO), and MLB/Rg1 group (5 mg/kg MLB/Rg1). Reperfusion was achieved by removing the filament and administering alteplase at 3 h after middle cerebral artery occlusion. Simultaneously with reperfusion, drugs were administered via tail vein immediately. The second MLB/Rg1 administration was performed at 24 h after reperfusion, the third MLB/Rg1 administration was performed at 48 h after reperfusion, and the fourth MLB/Rg1 administration was performed at 70 h after reperfusion. The 7 h after reperfusion was used as experimental endpoint and sampled. The MLB/Rg1 dose was 5 mg/kg. The Model group received physiological saline injection at the same time points and alteplase when reperfusion. In the Sham group, only the common carotid artery was isolated, ligated at the proximal end with a suture, and the wound was immediately closed. Administration times were the same as other groups, with physiological saline given.$Edema volume = infarcted hemisphere volume / Contralateral hemisphere volume$

To ensure objectivity, the experiment was conducted in a double-blind manner. The experimenters prepared the test substances one day before the start of the experiment and aliquoted them. A researcher not involved in this project replaced the test substance names with group codes, and blinding was not broken until all experiments were completed and conclusions were drawn.

### 1. MLB/Rg1 Improves Survival Rate in Reperfusion Mice

This experiment used a mouse cerebral ischemia-reperfusion model prepared by the filament method. The withdrawal of the filament simulates the reperfusion process of mechanical thrombectomy. Over time, reperfusion mice continued to die. The 72-hour mortality rate of tMCAO mice exceeded 50%, reaching 45.6%, while MLB/Rg1 significantly improved the 72-hour survival rate, with a survival rate as high as 90% (90% versus 45.6%, P<0.05) (Figure 26).

### 2. MLB/Rg1 Improves Edema in Reperfusion Mice

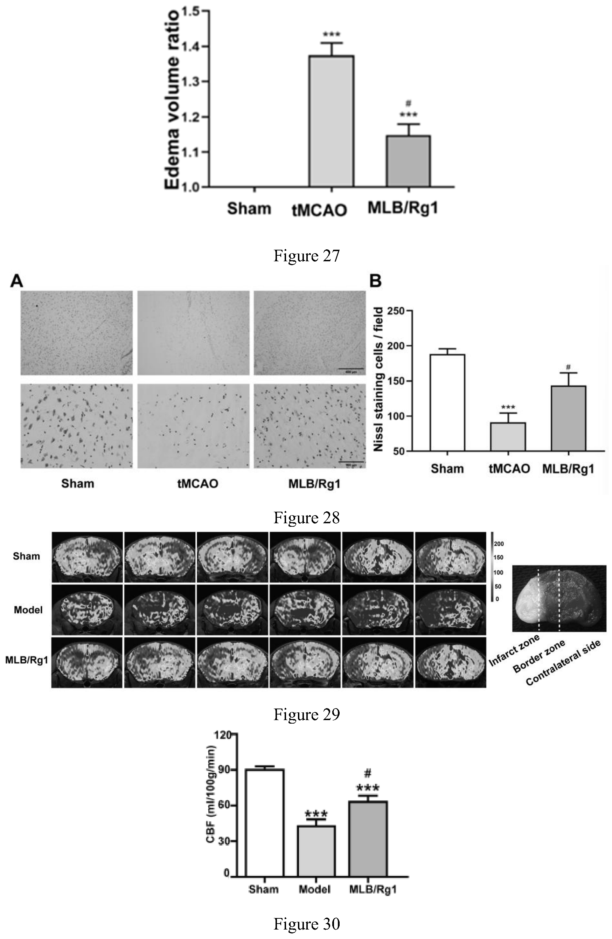

As expected, with the occurrence of infarction, the brain sections of reperfusion mice showed varying degrees of swelling, manifested as an increased ratio of infarct side to normal side volume. MLB/Rg1 significantly reduced the volume of brain edema. After weighing the mouse brain tissue, drying it, and weighing again, the water content of the brain tissue was calculated. It was found that the brain water content of the model group was significantly increased compared with the Sham group, indicating edema. Compared with the model group, MLB/Rg1 treatment reduced brain edema in tMCAO mice (Figure 27).

### 3. MLB/Rg1 Increases Neuron Number

The brain is the highest center for regulating body movement. Damage to the brain may lead to decreased body motor balance and stimulus-induced muscle movement. Damage to brain neurons can severely affect the neurological behavioral function of mice. Based on the finding that MLB/Rg1 can improve neurological behavioral function in stroke mice, Nissl staining was performed on brain tissue sections to determine whether MLB/Rg1 can protect nerve cells by counting the number of Nissl bodies.

In the Nissl-stained brain tissue images, the same location in the cerebral cortex of each image was selected for quantification. The number of Nissl-positive cells in the cortex after modeling was significantly reduced compared with the Sham group (P < 0.001). However, the number of Nissl-positive cells in the MLB/Rg1 group was significantly higher than that in the tMCAO group (P < 0.05) (Figure 28). This result corresponds to the behavioral data above, demonstrating that MLB/Rg1 improves neurological behavior in mice by protecting brain neurons.

### 4. MRI Reveals that MLB/Rg1 Increases Cerebral Blood Flow

Magnetic resonance imaging (MRI) is a sufficiently sensitive clinical diagnostic tool that can detect abnormalities in brain tissue and its surrounding environment. An 11.7 T small animal MRI system can achieve non-invasive detection of multiple parameters. In this study, an 11.7 T small animal MRI system was used to evaluate cerebral blood flow. Based on the distance from the infarct area, brain tissue was divided into the infarct core zone, border zone, and contralateral side, and cerebral blood flow (CBF) in the three regions was evaluated (Figure 29).

By measuring CBF, MLB/Rg1 significantly improved CBF in the infarct border zone. The results are shown in Figure 30. The CBF value of the MLB/Rg1 group was 63.92 ± 10.73 ml/100g/min, while that of the Model group was only 43.36 ± 11.24 ml/100g/min, with a significant difference between the two groups (P<0.05).

### Example 11: Synergistic Protective Effect of MLB/Rg1 on the Subacute Phase of Stroke

Fifty male SD rats with normal behavioral evaluations were randomly divided into 5 groups based on body weight. The body weights of the animals after grouping were tested for differences to ensure no differences among groups.

| Group (n=10) | Injection Reagent |
|---|---|
| Normal control group | 0.9% Sodium Chloride Injection |
| Model control group | 0.9% Sodium Chloride Injection |
| 5 mg/kg MLB/Rg1 group | Magnesium salvianolate B/Ginsenoside Rg1 (5:2) |
| MLB group | 3.57 mg/kg MLB |
| Rg1 group | 1.43 mg/kg Rg1 |

The administration volume for the normal control, model control, 5 mg/kg MLB/Rg1 (5:2), MLB, and Rg1 groups was 5 ml/kg, with 0.9% Sodium Chloride Injection as the vehicle. This experiment was conducted in a double-blind manner. This study used a rat reperfusion cerebral ischemia model, with blood flow reperfusion performed at 24 hafter middle cerebral artery occlusion. That is, the reperfusion time window was extended to 24 hours, which is a very stringent research system. Drugs were administered immediately after reperfusion performed at 24 h after modeling, followed by tail vein injection on day 1, 2, 3, 4, 5, and 6 after reperfusion according to the grouping and dosing table, with a total of seven administrations. To meet the energy requirements of animals unable to eat due to modeling, 4 ml of enteral nutrition suspension was administered by gavage twice daily after modeling. Tissue samples were collected 7 days after reperfusion to evaluate the subacute protective effect of extending the reperfusion time window to 24 hours on stroke.

### 1. MLB/Rg1 Significantly Extends the Reperfusion Time Window and Has a Significant Synergistic Effect in Reducing Infarct Volume

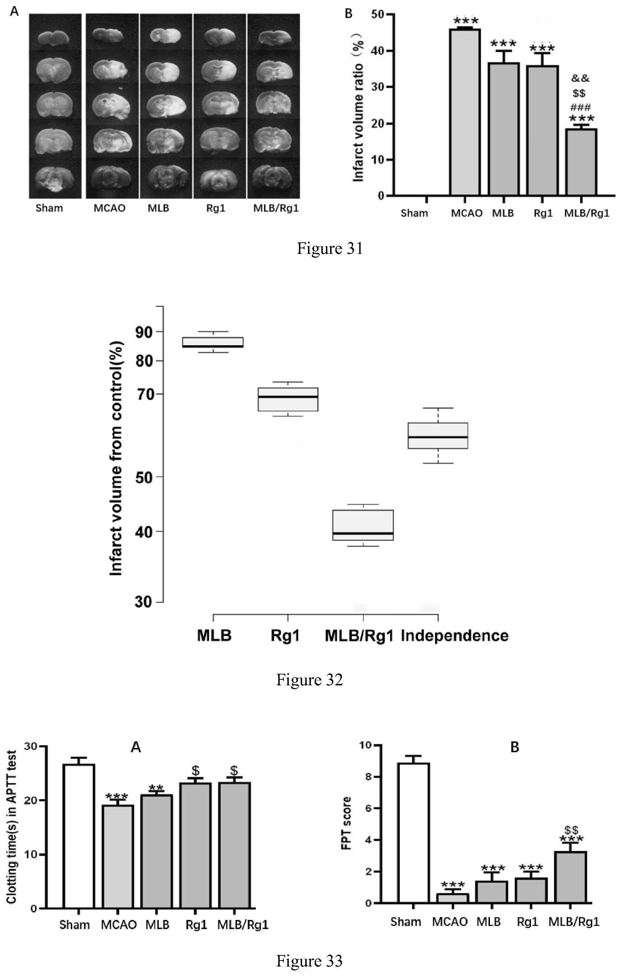

TTC staining was used to evaluate infarct volume (Figure 31A). Quantitative results of infarct volume showed that compared with the MCAO model group (46.07 ± 0.28%), the MLB alone group (39.51 ± 0.65%) and the Rg1 alone group (37.67 ± 0.88%) showed a trend of reducing infarct volume; the MLB/Rg1 group significantly reduced infarct volume compared with the model group (20.78 ± 0.69 vs 46.07 ± 0.28%, p<0.001), and also significantly reduced infarct volume compared with MLB alone (p<0.01) and Rg1 alone (p<0.01) (Figure 31B).

This study was designed according to the requirements of the Bliss independent statistical model, plotting the percentage of infarct volume for each treatment group relative to the MCAO group. The data of each group conformed to a normal distribution after the Kolmogorov-Smirnov test and met homogeneity of variance after the F-test. Relative to the MCAO group, the infarct volume percentage of the MLB/Rg1 group was 40.66 ± 0.88%, that of the MLB group was 85.77 ± 0.87%, and that of the Rg1 group was 68.75 ± 1.13%. According to the Bliss independent statistical model formula, the theoretical effect of the combination calculated based on the MLB and Rg1 groups, i.e., the infarct volume percentage of the Independence group was 59.04 ± 1.53%. Compared with the Independence group, the MLB/Rg1 group significantly reduced infarct volume (P < 0.01). Therefore, Bliss independent statistical analysis indicated that MLB and Rg1 have a significant synergistic effect in reducing infarct volume (Figure 32).

### 2. MLB/Rg1 Significantly Extends the Reperfusion Time Window, Delays Coagulation Time, and Improves Neurological Behavior

Blood was collected at day 7 after reperfusion for testing. Compared with the Sham group, the APTT values of the MCAO and MLB groups showed a significant decrease, indicating a significant shortening of coagulation time; compared with the MCAO group, the APTT values of the Rg1 and MLB/Rg1 groups showed a significant increase, indicating a prolongation of coagulation time. These results suggest that Rg1 and MLB/Rg1 have a certain anticoagulant effect in vivo. * represents compared with Sham group, *p<0.05, **p<0.01, ***p<0.001; $ represents compared with MCAO group, $p<0.05, $$p<0.01, $$$p<0.001 (Figure 33A).

Animal behavior was evaluated by the FPT test. MLB/Rg1 showed a significant difference compared with the MCAO group, indicating that MLB/Rg1 significantly improved animal behavioral ability. * represents compared with Sham group p<0.05, **p<0.01, ***p<0.001; $ represents compared with MCAO group, $p<0.05, $$p<0.01, $$$p<0.001 (Figure 33B).

### 3. MLB/Rg1 Significantly Extends the Reperfusion Time Window, Delays Coagulation Time, and Improves Neurological Behavior

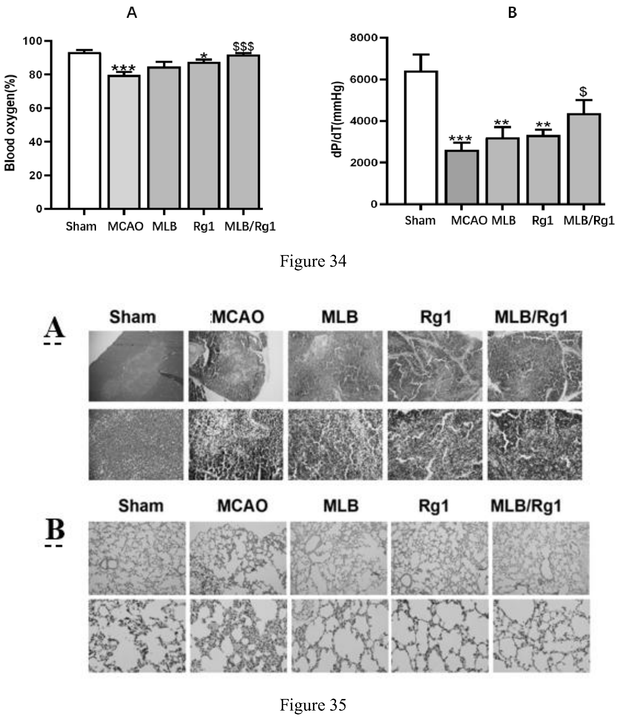

According to the blood oxygen measurement results, compared with the Sham group, the MLB and MCAO groups showed a significant decrease; compared with the MCAO group, the blood oxygen content of the MLB/Rg1 group was significantly increased. * represents compared with Sham group, p<0.05, **p<0.01, ***p<0.001; $ represents compared with MCAO group, p<0.05, $$p<0.01, $$$p<0.001 (Figure 34A).

dp/dt represents cardiac contractile function. Compared with the Sham group, the cardiac contractile functions of the MLB, MCAO, and Rg1 groups were significantly decreased, while the MLB/Rg1 group showed a significant recovery compared with the MCAO group. * represents compared with Sham group, p<0.05, **p<0.01, ***p<0.001; $ represents compared with tMCAO group, $p<0.05, $$p<0.01, $$$p<0.001 (Figure 34B).

### 4. MLB/Rg1 Significantly Extends the Reperfusion Time Window and Reduces Multi-Organ Damage after Stroke

Multiple organ damage may occur after stroke, leading to poor patient prognosis. Thymus was evaluated by HE staining. In the Sham group, the thymic medulla was larger, and the boundary between the medulla and cortex was clear and distinct. In the MCAO group, the thymic tissues in all groups showed atrophy with shrinkage of the medullary portion, obvious disorganization in medulla and cortex, blurred boundaries between the medulla and cortex, and increased fragmented tissues (Figure 35A).

Lung tissues were evaluated by HE staining. In the Sham group, lung cells were arranged neatly, alveolar walls were intact, and no red blood cell extravasation or lymphocyte infiltration was found in the alveoli and trachea. In the MCAO group, red blood cells were found in the alveoli, indicating bleeding in lung tissue. In addition, red blood cell deposition was found in the blood vessels of the MCAO group, with the highest proportion of pulmonary embolism (Figure 35B). MLB/Rg1 treatment alleviated these conditions to varying degrees.

Reperfusion therapy for cerebral ischemic diseases, such as stroke, aims to restore cerebral blood flow as soon as possible to reduce brain damage caused by ischemia. The wider the time window for reperfusion therapy, the more time patients have to receive treatment. Typically, the reperfusion therapy time window for acute ischemic stroke is within 4.5 hours of onset. The difficulty and risk of treatment increase over time: Early treatment (0-4.5 hours): within this time period, intravenous thrombolysis is one of the most effective treatments; Mid-term treatment (4.5-6 hours or longer): in this stage, in addition to intravenous thrombolysis, interventional therapies such as mechanical thrombectomy may be considered. However, as treatment time is delayed, the risk of hemorrhagic transformation also increases. Late treatment (more than 6 hours): although studies have shown that under specific conditions, such as selecting potentially benefiting patients through advanced imaging techniques, reperfusion therapy can be implemented beyond the conventional time window, overall, the efficacy decreases, and the risk of complications increases. In conclusion, regardless of the method, earlier treatment generally leads to better outcomes. As time extends, the complexity and risk of treatment increase accordingly.

The above results indicate that the MLB/Rg1 combination still has a significant synergistic protective effect in animals undergoing reperfusion at 24 h after middle cerebral artery occlusion, suggesting that it can extend the reperfusion time window for patients with cerebral ischemia, potentially improving the efficacy of late-stage treatment for these patients, reducing complications, and enhancing motor ability, which is crucial for improving patients' self-care ability and quality of life.

All documents mentioned in the present invention are incorporated by reference into this application, as if each document were individually cited as a reference. Furthermore, it should be understood that after reading the above teachings of the present invention, those skilled in the art can make various changes or modifications to the present invention, and these equivalent forms are also within the scope defined by the appended claims of this application.

## Claims

1. A pharmaceutical composition, wherein the active ingredients of the pharmaceutical composition comprise (a) and (b):
(a) a first active ingredient, which is a pharmaceutically acceptable salt of salvianolic acid; and
(b) a second active ingredient, which is ginsenoside, or a pharmaceutically acceptable salt thereof;
wherein the pharmaceutically acceptable salt of salvianolic acid is selected from the group consisting of: magnesium salvianolate B, calcium salvianolate B, magnesium salvianolate A, sodium rosmarinate, or combinations thereof;
the ginsenoside is selected from the group consisting of: ginsenoside Rg1, ginsenoside Rh2, ginsenoside Rb1, or combinations thereof;
and the weight ratio of the first active ingredient to the second active ingredient is 1:(0.1-10), wherein the weight ratio is calculated based on the pharmaceutically acceptable salt of salvianolic acid and ginsenoside.

2. The pharmaceutical composition according to claim 1, **characterized in that** the first active ingredient is selected from the group consisting of: magnesium salvianolate B, calcium salvianolate B, or a combination thereof;
and the second active ingredient is ginsenoside Rg1, or a pharmaceutically acceptable salt thereof.

3. The pharmaceutical composition according to claim 1, **characterized in that** the first active ingredient is magnesium salvianolate B; and the second active ingredient is ginsenoside Rg1;
and the weight ratio of the first active ingredient to the second active ingredient is 1:(0.1-10), wherein the weight ratio is calculated based on magnesium salvianolate B and ginsenoside Rg1.

4. The pharmaceutical composition according to any one of claims 1-3, **characterized in that** the weight ratio of the first active ingredient to the second active ingredient is 1:(0.2-5).

5. The pharmaceutical composition according to any one of claims 1-3, **characterized in that** the weight ratio of the first active ingredient to the second active ingredient is 1:(0.38-0.42).

6. The pharmaceutical composition according to any one of claims 1-3, **characterized in that** the weight ratio of the first active ingredient to the second active ingredient is 1:0.4.

7. The pharmaceutical composition according to any one of claims 1-3, **characterized in that** the dosage form of the pharmaceutical composition is selected from the group consisting of: liquid preparations, solid preparations, gaseous dosage forms, and semi-solid dosage forms.

8. The pharmaceutical composition according to any one of claims 1-3, **characterized in that** the dosage form is selected from the group consisting of: injections, oral preparations, sublingual preparations, respiratory tract administration preparations, skin administration preparations, and mucosal administration preparations.

9. The pharmaceutical composition according to any one of claims 1-3, **characterized in that** the dosage form is selected from the group consisting of: injection solutions, powder for injection, capsules, tablets, pills, powders, granules, syrups, oral liquids, tinctures, lozenges and drops.

10. A pharmaceutical composition for oral administration, **characterized in that** the active ingredients of the pharmaceutical composition comprise (a) and (b):
(a) a first active ingredient, which is a pharmaceutically acceptable salt of salvianolic acid B; and
(b) a second active ingredient, which is ginsenoside, or a pharmaceutically acceptable salt thereof;
wherein the pharmaceutically acceptable salt of salvianolic acid B is selected from the group consisting of: magnesium salvianolate B, calcium salvianolate B, or combinations thereof;
and the weight ratio of the first active ingredient to the second active ingredient is 1:(0.1-10), wherein the weight ratio is calculated based on the pharmaceutically acceptable salt of salvianolic acid B and ginsenoside.

11. The pharmaceutical composition according to claim 10, **characterized in that** the first active ingredient is magnesium salvianolate B; and the second active ingredient is ginsenoside Rg1;
and the weight ratio of the first active ingredient to the second active ingredient is 1:(0.1-10), wherein the weight ratio is calculated based on magnesium salvianolate B and ginsenoside Rg1.

12. The pharmaceutical composition according to claim 10 or 11, **characterized in that** the dosage form of the pharmaceutical composition is an orally disintegrating tablet, mucoadhesive tablet, oral dissolving film, orally disintegrating granules, powders, sublingual tablet, buccal tablet, chewable tablet, effervescent tablet, or solution.

13. Use of the pharmaceutical composition according to any one of claims 1-12 in the preparation of a medicament for preventing and/or treating brain diseases.

14. The use according to claim 13, **characterized in that** the brain disease is cerebrovascular disease or cerebral ischemic disease, preferably, the cerebrovascular disease or cerebral ischemic disease is selected from the group consisting of: ischemic stroke, cerebral arteriosclerosis, cerebral circulation insufficiency, cerebral hemorrhage, cerebral edema, or combinations thereof.

15. The use according to claim 13, **characterized in that** the brain disease is senile dementia, vascular dementia, brain atrophy, dementia caused by brain atrophy of unknown etiology, as well as neuronal cell loss, behavioral disorders, sensory disorders caused by chronic brain injury, or combinations thereof.

16. The use according to claim 13, **characterized in that** the brain disease is a neurodegenerative disease, preferably, the neurodegenerative disease is selected from the group consisting of: Alzheimer's disease (AD), hippocampal sclerosis, Parkinson's disease, amyotrophic lateral sclerosis, multiple system atrophy, progressive supranuclear palsy, or combinations thereof.

17. The use according to claim 13, **characterized in that** the preventing and/or treating brain diseases is selected from the group consisting of: inhibiting white matter atrophy, inhibiting hippocampal atrophy, inhibiting lateral ventricle enlargement, inhibiting striatal atrophy, or combinations thereof.

18. The use according to claim 13, **characterized in that** the preventing and/or treating brain diseases is selected from the group consisting of: inhibiting neuronal cell loss in white matter, inhibiting neuronal cell loss in hippocampus, inhibiting lateral ventricle enlargement, inhibiting neuronal cell loss in striatum, or combinations thereof.

19. The use according to claim 13, **characterized in that** the patient is in the acute, subacute, or chronic phase after the onset of ischemic stroke.

20. The use according to claim 13, **characterized in that** the pharmaceutical composition is used for preventing or treating cardiac injury, pulmonary injury, thymic injury, renal injury, or combinations thereof caused by ischemic stroke;
systemic hypoxia caused by ischemic stroke, or secondary injury due to hypoxia; and/or
reperfusion injury after ischemic stroke, such as cardiac injury, pulmonary injury, thymic injury, renal injury, or combinations thereof caused by reperfusion.
